(19) **Europäisches Patentamt / European Patent Office / Office européen des brevets**

(11) **EP 2 741 769 B1**

(12) **EUROPEAN PATENT SPECIFICATION**

(45) Date of publication and mention
of the grant of the patent:
**02.01.2019 Bulletin 2019/01**

(21) Application number: **12780530.7**

(22) Date of filing: **10.08.2012**

(51) Int Cl.:
***A61K 39/00*** (2006.01)

(86) International application number:
**PCT/IB2012/001795**

(87) International publication number:
**WO 2013/021279 (14.02.2013 Gazette 2013/07)**

(54) **HIGHLY GALACTOSYLATED ANTIBODIES**

HOCH GALACTOSYLIERTE ANTIKÖRPER

ANTICORPS FORTEMENT GALACTOSYLÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.08.2011 US 201161521996 P
06.12.2011 US 201161567364 P**

(43) Date of publication of application:
**18.06.2014 Bulletin 2014/25**

(73) Proprietor: **Laboratoire Français du
Fractionnement et des
Biotechnologies
91940 Les Ulis (FR)**

(72) Inventors:
• **FAID, Valegh
F-91940 Les Ulis (FR)**
• **CHEVREUX, Guillaume
F-75009 Paris (FR)**

(74) Representative: **Chajmowicz, Marion et al
Becker & Associés
25, rue Louis Le Grand
75002 Paris (FR)**

(56) References cited:
**WO-A1-2007/115813    WO-A2-2007/005786**

**WO-A2-2008/028686    FR-A1- 2 861 080**

• **POLLOCK D P ET AL: "Transgenic milk as a
method for the production of recombinant
antibodies", JOURNAL OF IMMUNOLOGICAL
METHODS, ELSEVIER SCIENCE PUBLISHERS
B.V.,AMSTERDAM, NL, vol. 231, no. 1-2, 10
December 1999 (1999-12-10), pages 147-157,
XP004187641, ISSN: 0022-1759, DOI:
10.1016/S0022-1759(99)00151-9**
• **GRAMER M J ET AL: "Modulation of Antibody
Galactosylation Through Feeding of Uridine,
Manganese Chloride, and Galactose",
BIOTECHNOLOGY AND BIOENGINEERING,
WILEY & SONS, HOBOKEN, NJ, US, vol. 108, no.
7, 1 July 2011 (2011-07-01), pages 1591-1602,
XP002688515, ISSN: 0006-3592, DOI:
10.1002/BIT.23075 [retrieved on 2011-02-18]**
• **RAJU T S ET AL: "Species-specific variation in
glycosylation of IgG: evidence for
species-specific sialylation and branch-specific
galactosylation and importance for engineering
recombinant glycoprotein therapeutics",
GLYCOBIOLOGY, OXFORD UNIVERSITY PRESS,
US, vol. 10, no. 5, 1 January 2000 (2000-01-01),
pages 477-486, XP002964086, ISSN: 0959-6658,
DOI: 10.1093/GLYCOB/10.5.477**

Remarks:
The file contains technical information submitted after
the application was filed and not included in this
specification

EP 2 741 769 B1

**Description**

FIELD OF THE INVENTION

The present invention relates to highly galactosylated antibodies, methods of their production as well as methods of their use.

BACKGROUND OF THE INVENTION

Therapeutic antibodies are being used in the treatment of a number of disorders, including cancer.

French patent application 2 861 080 teaches how to produce populations of antibodies that have an optimal ratio of fucosylation to glycosylation. International patent application WO2007/048077 describes antibodies with enhanced antibody-dependent cellular cytotoxicity activity, preferably with low-fucosylation profiles.

However, there is a need for therapeutic antibodies with improved properties.

SUMMARY OF THE INVENTION

In one aspect, the disclosure provides compositions comprising populations of antibodies that are highly galactosylated. The antibodies have been produced transgenically in goat mammary gland epithelial cells.

More particularly, the invention provides a method for producing a highly galactosylated population of antibodies, comprising transgenically expressing a population of antibodies in mammary gland epithelial cells of a goat such that a highly galactosylated population of antibodies is produced, wherein the level of galactosylation of the antibodies in the population is at least 70%.

In some embodiments, the highly galactosylated antibodies are also highly fucosylated. In some embodiments, the highly galactosylated antibodies show both high complement dependent cytotoxicity (CDC) activity and high antibody-dependent cellular cytotoxicity (ADCC) activity. In one aspect, the disclosure provides a method for producing populations of antibodies that are highly galactosylated.

In one aspect, the disclosure provides a composition comprising a population of antibodies wherein the population of antibodies is highly galactosylated. According to the invention, the antibodies in the population are transgenically produced in goat mammary gland epithelial cells. In some embodiments, the antibodies in the population are directed to the same antigen epitope.

In some embodiments, the antibodies in the population are encoded by the same nucleic acid sequence. The level of galactosylation of the antibodies in the population is at least 70%. In some embodiments, the population of antibodies comprises antibodies that comprise mono-galactosylated N-glycans.

In one aspect, the disclosure provides a composition comprising a population of antibodies wherein the level of galactosylation of the antibodies in the population is at least 70%, wherein the population of antibodies comprises antibodies that comprise mono-galactosylated N-glycans, and wherein antibodies in the population are encoded by the same nucleic acid sequence.

In some embodiments of any of the compositions provided herein, the level of galactosylation of the antibodies in the population is at least 80%. In some embodiments of any of the compositions provided herein, the level of galactosylation of the antibodies in the population is at least 90%. In some embodiments of any of the compositions provided herein, the level of fucosylation of the antibodies in the population is at least 80%. In some embodiments of any of the compositions provided herein, the level of fucosylation of the antibodies in the population is at least 90%.

In one aspect, the disclosure provides a composition comprising a population of antibodies wherein the ratio of the level of galactosylation of the antibodies in the population to the level of fucosylation of the antibodies in the population is between 0.8 and 1.2, wherein the population of antibodies comprises antibodies that comprise mono-galactosylated N-glycans, and wherein antibodies in the population are encoded by the same nucleic acid sequence.

In some embodiments of any of the compositions provided herein, the population of antibodies comprises antibodies that comprise bi-galactosylated N-glycans. In some embodiments of any of the compositions provided herein, the population of antibodies comprises antibodies that comprise both mono-galactosylated N-glycans and bi-galactosylated N-glycans. In some embodiments of any of the compositions provided herein, at least 35% of the antibodies in the population comprise bi-galactosylated N-glycans and at least 5% of the antibodies in the population comprise mono-galactosylated N-glycans.

According to the invention, the population of antibodies are transgenically produced in the mammary gland epithelial cells of a goat engineered to express the antibodies..

In some embodiments of any of the compositions provided herein, the composition further comprises milk.

In some embodiments of any of the compositions provided herein, the antibodies of the population of antibodies have an increased level of complement dependent cytotoxicity (CDC) activity when compared to a population of antibodies not produced in mammary gland epithelial cells. In some embodiments, the antibodies of the population of antibodies not produced in mammary gland epithelial cells are produced in cell culture. In some embodiments, the antibodies of the population of antibodies not produced in mammary gland epithelial cells are low-galactose antibodies. In some embodiments, the low-galactose antibodies are Rituxan.

In some embodiments of any of the compositions provided herein, the antibodies of the population of antibodies have an increased level of the antibody-dependent cellular cytotoxicity (ADCC) activity when compared to a population of antibodies not produced in mammary gland epithelial cells. In some embodiments, the antibodies of the population of antibodies not produced in mammary gland epithelial cells are produced in cell culture. In some embodiments, the antibodies of the population of antibodies not produced in mammary gland epithelial cells are low-galactose antibodies. In some embodiments, the low-galactose antibodies are Rituxan.

In some embodiments of any of the compositions provided herein, the antibodies in the population are chimeric, humanized or fully human antibodies. In some embodiments, the antibodies in the population are full-length antibodies. In some embodiments, the antibodies in the population comprise a heavy chain and a light chain. In some embodiments, the antibodies in the population are anti-CD20 antibodies. In some embodiments, the light chain and heavy chain of the antibodies in the population are encoded by nucleic acid sequences as set forth in SEQ ID NO:1 and SEQ ID NO:2.

In some embodiments of any of the compositions provided herein, the composition further comprises a pharmaceutically acceptable carrier.

In one aspect, the disclosure provides a method comprising administering any of the compositions provided herein to a subject in need thereof. In some embodiments, the subject has cancer. In some embodiments, the cancer is B-cell lymphoma. In some embodiments, the subject has an immune disorder.

In one aspect, the disclosure provides a method for producing a highly galactosylated population of antibodies comprising producing the population of antibody in mammary gland epithelial cells such that a highly galactosylated population of antibodies is produced. In some embodiments, the method further comprises collecting the population of antibodies produced.

In one aspect, the disclosure provides a method for producing a highly galactosylated population of antibodies, comprising collecting a highly galactosylated population of antibodies produced in mammary gland epithelial cells engineered to express the antibodies.

In some embodiments of any of the methods provided herein, the method further comprises determining the CDC activity of the population of antibodies.

In some embodiments of any of the methods provided herein, the method further comprises comparing the CDC activity of the population of antibodies to a population of antibodies not produced in mammary gland epithelial cells. In some embodiments, the antibodies of the population of antibodies not produced in mammary gland epithelial cells are produced in cell culture.

In some embodiments of any of the methods provided herein, the method further comprises determining the ADCC activity of the population of antibodies.

In some embodiments of any of the methods provided herein, the method further comprises comparing the ADCC activity of the population of antibodies to a population of antibodies not produced in mammary gland epithelial cells. In some embodiments, the antibodies of the population of antibodies not produced in mammary gland epithelial cells are produced in cell culture.

In some embodiments of any of the methods provided herein, the method further comprises determining the level of galactosylation of the population of antibodies.

In some embodiments of any of the methods provided herein, the mammary gland epithelial cells are in culture and are transfected with a nucleic acid that comprises a sequence that encodes the antibody.

In the methods provided herein, the mammary gland epithelial cells are in a goat engineered to express a nucleic acid that comprises a sequence that encodes the antibody in its mammary gland.

In the methods provided herein, the level of galactosylation of the antibodies in the population is at least 70%. In some embodiments, the level of galactosylation of the antibodies in the population is at least 80%. In some embodiments, the level of galactosylation of the antibodies in the population is at least 90%.

In some embodiments of any of the methods provided herein, the level of fucosylation of the antibodies in the population is at least 80%. In some embodiments, the level of fucosylation of the antibodies in the population is at least 90%.

In some embodiments of any of the methods provided herein, the ratio of the level of galactosylation of the antibodies in the population to the level of fucosylation of the antibodies in the population is between 0.8 and 1.2.

In some embodiments of any of the methods provided herein, the population of antibodies comprises antibodies that comprise mono-galactosylated N-glycans

In some embodiments of any of the methods provided herein, the population of antibodies comprises antibodies that comprise bi-galactosylated N-glycans.

In some embodiments of any of the methods provided herein, the population of antibodies comprises antibodies that comprise both mono-galactosylated N-glycans and bi-galactosylated N-glycans.

In some embodiments of any of the methods provided herein, at least 35% of the antibodies in the population comprise bi-galactosylated N-glycans and at least 5% of the antibodies in the population comprise mono-galactosylated N-glycans.

In some embodiments of any of the methods provided herein, the method further comprises purifying the population of antibodies such that the level of galactosylation of the antibodies in the population is at least 70%. In some embodiments, the method further comprises purifying the population of antibodies such that the level of galactosylation of the antibodies in the population is at least 80%. In some embodiments, the method further comprises purifying the population of antibodies such that the level of galactosylation of the antibodies in the population is at least 90%.

In some embodiments of any of the methods provided herein, the method further comprises purifying the population of antibodies such that the level of fucosylation of the antibodies in the population is at least 80%. In some embodiments, the method further comprises purifying the population of antibodies such that the level of fucosylation of the antibodies in the population is at least 90%.

In some embodiments of any of the methods provided herein, the method further comprises purifying the population of antibodies such that the ratio of the level of galactosylation of the antibodies in the population to the level of fucosylation of the antibodies in the population is between 0.8 and 1.2.

In some embodiments of any of the methods provided herein, the population of antibodies comprises antibodies that comprise mono-galactosylated N-glycans.

In some embodiments of any of the methods provided herein, the population of antibodies comprises antibodies that comprise bi-galactosylated N-glycans.

In some embodiments of any of the methods provided herein, the population of antibodies comprises antibodies that comprise both mono-galactosylated N-glycans and bi-galactosylated N-glycans.

In some embodiments of any of the methods provided herein, at least 35% of the antibodies in the population comprise bi-galactosylated N-glycans and at least 5% of the antibodies in the population comprise mono-galactosylated N-glycans.

In some embodiments of any of the methods provided herein, the antibodies in the population are directed to the same antigen epitope.

In some embodiments of any of the methods provided herein, the antibodies in the population are anti-CD20 antibodies. In some embodiments, the light chain and heavy chain of the antibodies in the population are encoded by nucleic acid sequences as set forth in SEQ ID NO:1 and SEQ ID NO:2.

In one aspect, the disclosure provides mammary gland epithelial cells that express any of the population of antibodies provided herein

In one aspect, the disclosure provides a transgenic goat comprising any of the mammary gland epithelial cells provided herein.

BRIEF DESCRIPTION OF THE DRAWINGS

Figure 1 shows the sequences of the light chain (SEQ ID NO:1; Figure 1A) and the heavy chain (SEQ ID NO:2; Figure 1B) of Tg20.

Figure 2 shows the molecular strategy for generating the transgenic anti CD20 antibody (TG20).

Figure 3 shows an SDS-PAGE under non-reducing and reducing conditions of Tg20, a low fucose reference antibody, and a MW standard (Std); Coomassie blue stained (left panel) and silver stained (right panel); apparent molecular weight in kDa.

Figure 4 shows the NP-HPLC profile of 2-AB labeled PNGase F-released N-glycans from Tg20.

Figure 5 shows a size exclusion profile of Tg20.

Figure 6 shows the experimental conditions of the Whole blood assay.

Figure 7 shows a method for the generation of TG20 expressing goats.

Figure 8 shows a comparison of functionalities of Tg20 and Rituxan.

Figure 9 shows the results of an ADCC/CD16 assay for Tg20 and Rituxan (RTX).

Figure 10 shows the pharmacokinetic profile in cynomolgus monkeys of Tg20 and RTX.

Figure 11 shows the pharmacological activity in cynomolgus monkeys of Tg20 and RTX.

Figures 12A and 12B show the individual kinetics of the percentage of residual B lymphocytes (CD45+/CD3-/CD40+) in blood.

Figure 13 shows the kinetics of the mean percentage of residual B lymphocytes (CD45+/CD3-/CD40+) in blood, for all study animals.

Figure 14 shows the kinetics of the mean percentage of residual B lymphocytes (CD45+/CD3-/CD40+) in blood, for recovery animals only.

Figure 15 shows the individual kinetics of the relative percentage of B lymphocytes (CD45+/CD3-/CD40+) in lymph nodes.

Figure 16 shows the kinetics of the mean relative percentage of B lymphocytes (CD45+/CD3-/CD40+) in lymph nodes, for all study animals.

Figure 17 shows the individual kinetics of the residual percentage of B lymphocytes (CD45+/CD3-/CD40+) in lymph nodes, when compared to the control group mean.

Figure 18 shows the kinetics of the mean residual percentage of B lymphocytes (CD45+/CD3-/CD40+) in lymph nodes, for all study animals, when compared to the control group mean.

DETAILED DESCRIPTION

In one aspect, the disclosure provides compositions of populations of antibodies that are highly galactosylated. In some embodiments, the antibodies have been produced transgenically. In some embodiments, the highly galactosylated antibodies are also highly fucosylated. In some embodiments, the highly galactosylated antibodies show both high complement dependent cytotoxicity (CDC) activity and high antibody-dependent cellular cytotoxicity (ADCC) activity. In one aspect, the disclosure provides methods for producing populations of antibodies that are highly galactosylated.

*Glycosylation*

In one aspect, the disclosure provides compositions of populations of antibodies that are highly galactosylated. In the invention, the populations of antibodies that are highly galactosylated are transgenically produced in goat mammary gland epithelial cells. In some embodiments, the antibodies in the population of antibodies are directed to the same antigen epitope. In some embodiments, the antibodies in the population of antibodies are encoded by the same nucleic acid sequence.

In one aspect, the disclosure provides compositions comprising a population of antibodies that are highly galactosylated, wherein the population of antibodies comprises mono-galactosylated N-glycans. In some embodiments, the population of antibodies is transgenically produced. In some embodiments, the antibodies in the population of antibodies are encoded by the same nucleic acid sequence.

Antibodies can be glycosylated at the Fc-gamma glycosylation site (Asn 297 of the Fc region). A variety of glycosylation patterns has been observed at the Fc gamma glycosylation site and the oligosaccharides found at this site include galactose, N-acetylglucosamine (GlcNac), mannose, sialic or N-acetylneuraminic acid (NeuAc) and fucose. The oligosaccharides found at these sites (N-glycans) all have a common core-structure, consisting of an N-acetylglucosamine (GlcNAc) attached to the asparagine, to which a second GlcNAc and three mannoses are attached. This core may carry a multitude of different glycan motifs. The most common type of N-glycans of plasma proteins is the complex type. In the biosynthetic route to this N-glycan type, several GlcNAc transferases attach GlcNAc residues to the mannoses of the glycan core, which can be further extended by galactose, sialic acid and fucose residues. Another group of N-glycans are the high-mannose glycoproteins, which are characterized by a high number (five or more) mannose attached to the second GlcNAc. Hybrid structures in which one of the biantennary arms is mannose substituted while the other arm is complex have also been found. Fucose residues are generally not found in the "arms" of the bi-antennary structure but are attached to the N-linked GlcNac.

The biosynthesis of N-glycans is not regulated by a template, as is the case with proteins, but is mainly dependent on the expression and activity of specific glycosyltransferases in a cell. Therefore, a glycoprotein, such as an antibody Fc domain, normally exists as a heterogeneous population of glycoforms which carry different glycans on the same protein backbone.

The glycosylation pattern can be determined by many methods known in the art. For example, methods of analyzing carbohydrates on proteins have been described in U.S. Patent Applications US 2006/0057638 and US 2006/0127950. The methods of analyzing carbohydrates on proteins are incorporated herein by reference.

A population of antibodies that is highly galactosylated is a population of antibodies wherein the level of galactosylation of the antibodies in the population is at least 50 %, at least 60%, at least 70%, at least 80%, at least 90%, up to 100% of galactosylation. In some embodiments of the population of antibodies that is highly galactosylated, the level of galactosylation of the antibodies in the population is at least 70%.

The level of galactosylation as used herein is determined by the following formula:

$$\frac{\sum_{i=1}^{n} (number\ of\ Gal)\ *\ (\%\ relative\ Area)}{\sum_{i=1}^{n} (number\ of\ A)\ *\ (\%\ relative\ Area)} * 100$$

wherein:

- n represents the number of analyzed N-glycan peaks of a chromatogram, such as a Normal-Phase High Performance Liquid Chromatography (NP HPLC) spectrum
- *"number of Gal"* represents the number of *Galactose* motifs on the antennae of the glycan corresponding to the peak, and
- *"number of A"* corresponds to the number of *N*-acetylglucosamine antennae of the glycan form corresponding to the peak, and
- *"% relative Area"* corresponds to % of the Area under the corresponding peak

Thus, the level of the level of galactosylation of the antibodies in a population can be determined by releasing the N-glycans from the antibodies, resolving the N-glycans on a chromatogram, identifying the N-glycan that corresponds to a specific peak, determining the peak intensity and applying the data to the formula provided above (See also the experimental section provided herein).

Antibodies that are galactosylated includes any antibody that has at least one galactose monosaccharide. Such antibodies include both antibodies that have a complex glycan motif on both arms of the "antenna" and antibodies that have only one arm with a complex glycan motif. Antibodies that include at least one galactose monosaccharide include antibodies with the N-glycans such as G1 (one galactose), G1F (one galactose, one fucose), G2 (two galactoses) and G2F (two galactoses, one fucose). In addition, the N-glycan that includes at least one galactose monosaccharide can be sialylated or not sialylated.

In some embodiments of the population of antibodies that is highly galactosylated, the population comprises antibodies that comprise mono-galactosylated N-glycans, which may or may not be sialylated, and corresponding in whole or in part to A2G1F form. In some embodiments of the population of antibodies that is highly galactosylated, at least 1%, at least 5%, at least 10%, at least 15%, at least 20%, at least 25%, at least 30%, at least 40%, at least 50%, at least 60%, at least 70%, at least 80%, at least 90%, up to 100% of the antibody N-glycans comprise mono-galactosylated N-glycans. In some embodiments of the population of antibodies that is highly galactosylated, at least 5% of the antibodies comprise mono-galactosylated N-glycans.

In some embodiments of the population of antibodies that is highly galactosylated, the population comprises antibodies that comprise mono-galactosylated N-glycans, which may or may not be sialylated and corresponding in whole or in part to A2G1F form, and antibodies that comprise bi-galactosylated N-glycans, which may or may not be sialylated, corresponding in whole or in part to A2G2F form. In some embodiments of the population of antibodies that is highly galactosylated, at least 1%, at least 5%, at least 10%, at least 15%, at least 20%, at least 25%, at least 30%, at least 40%, at least 50%, at least 60%, at least 70%, at least 80%, at least 90%, up to 99% of the antibody N-glycans comprise mono-galactosylated N-glycans, and at least 1%, at least 5%, at least 10%, at least 15%, at least 20%, at least 25%, at least 30%, at least 40%, at least 50%, at least 60%, at least 70%, at least 80%, at least 90%, up to 99% of the antibody N-glycans comprise bi-galactosylated N-glycans. In some embodiments of the population of antibody N-glycans that is highly galactosylated, at least 5% of the antibody N-glycans comprise mono-galactosylated N-glycans and at least 35% of the antibodies comprise bi-galactosylated N-glycans.

In some embodiments of the population of antibodies that is highly galactosylated, the population comprises antibodies that are highly fucosylated. A population of antibodies that is highly fucosylated is a population of antibodies wherein the level of fucosylation of the antibody N-glycans in the population is at least 50 %, at least 60%, at least 70%, at least 80%, at least 90%, up to 100% of fucosylation. In some embodiments in the population of antibodies that is highly galactosylated, the level of fucosylation of the antibody N-glycans is at least 80%.

The level of fucosylation as used herein is determined by the following formula:

$$\sum_{i=1}^{n} (number\ of\ Fucose)\ *\ (\%\ relative\ Area)$$

wherein:

- n represents the number of analyzed N-glycan peaks of a chromatogram, such as a Normal-Phase High Performance Liquid Chromatography (NP HPLC) spectrum, and
- *"number of Fucose"* represents the number of *Fucose* motifs on the glycan corresponding to the peak, and
- *"% relative Area"* corresponds to % of the Area under the corresponding peak containing the *Fucose* motif.

Antibodies that are fucosylated includes any antibody that has at least one fucose monosaccharide on its N-glycans.

In some embodiments, the population of antibodies disclosed herein relates to a population wherein the level of galactosylation of the antibody N-glycans in the population is at least 50% and the level of fucosylation of the antibodies in the population is at least 50%. In some embodiments, the population of antibodies disclosed herein relates to a population wherein the level of galactosylation of the antibody N-glycans in the population is at least 50%, and the level of fucosylation of the antibody N-glycans in the population is at least 60%, at least 70%, at least 80%, at least 90%, up to 100%. In some embodiments, the population of antibodies disclosed herein relates to a population wherein the level of galactosylation of the antibody N-glycans in the population is at least 60%, and the level of fucosylation of the antibody N-glycans in the population is at least 60%, at least 70%, at least 80%, at least 90%, up to 100%. In some embodiments, the population of antibodies disclosed herein relates to a population wherein the level of galactosylation of the antibody N-glycans in the population is at least 70%, and the level of fucosylation of the antibody N-glycans in the population is at least 60%, at least 70%, at least 80%, at least 90%, up to 100%. In some embodiments, the population of antibodies disclosed herein relates to a population wherein the level of galactosylation of the antibody N-glycans in the population is at least 80%, and the level of fucosylation of the antibody N-glycans in the population is at least 60%, at least 70%, at least 80%, at least 90%, up to 100%. In some embodiments, the population of antibodies disclosed herein relates to a population wherein the level of galactosylation of the antibody N-glycans in the population is at least 90%, and the level of fucosylation of the antibody N-glycans in the population is at least 60%, at least 70%, at least 80%, at least 90%, up to 100%. In some embodiments, the population of antibodies disclosed herein relates to a population wherein the level of galactosylation of the antibody N-glycans in the population is up to 100% and the level of fucosylation of the antibody N-glycans in the population is at least 60%, at least 70%, at least 80%, at least 90%, up to 100%.

In one aspect, the disclosure relates to a composition comprising a population of antibodies with a specific ratio of the percentage of antibody N-glycans in the population that are galactosylated at the Fc-gamma-glycosylation site to the percentage of antibody N-glycans in the population that are fucosylated at the Fc-gamma-glycosylation site. In some embodiments, the disclosure relates to a composition comprising a population of antibodies wherein the ratio of the level of galactosylation of the antibody N-glycans in the population to the level of fucosylation of the antibody N-glycans in the population has a specific ratio. In some embodiments, the population of antibodies comprises antibody N-glycans that comprise mono-galactosylated N-glycans, which may be or may not be sialylated and corresponding in whole or in part to A2G1F form. In some embodiments, the antibodies in the population of antibodies are directed to the same antigen epitope. In some embodiments, the antibodies in the population of antibodies are encoded by the same nucleic acid sequence. In some embodiments, the disclosure relates to a composition comprising a population of antibodies wherein the ratio of the level of galactosylation of the antibody N-glycans in the population to the level of fucosylation of the antibody N-glycans in the population is between 0.5 and 2, between 0.6 and 1.8, between 0.7 and 1.5, between 0.8 and 1.2, or between 0.9 and 1.1. In some embodiments, the disclosure relates to a composition comprising a population of antibodies wherein the ratio of the level of galactosylation of the antibody N-glycans in the population to the level of fucosylation of the antibody N-glycans in the population is between 0.8 and 1.2, for example 1.

*Production of highly galactosylated antibodies*

In one aspect, the disclosure provides compositions comprising populations of antibodies that are highly galactosylated. In some embodiments, the populations of antibodies that are highly galactosylated are transgenically produced. In some embodiments, the populations of antibodies are produced in cell culture. In some embodiments, the cell culture has been modified to increase the amount of antibody galactosylation, *e.g.,* by adding galactosyltransferases to the cell culture, or adding by genetic material to the cell that results in the increased productions of galactosyltransferases. In some embodiments, the populations of antibodies are produced in cell culture and subsequently modified by an *in vitro* biochemical reaction to attach (additional) galactose.

In some embodiments, the populations of antibodies that are highly galactosylated are transgenically produced. In some embodiments, the populations of antibodies are produced in mammary gland epithelial cells. In some embodiments, the populations of antibodies are produced in the mammary gland epithelial cells of a non-human mammal engineered to express the antibodies. In some embodiments, the non-human mammal is a goat, sheep, bison, camel, cow, pig, rabbit, buffalo, horse, rat, mouse or llama. In some embodiments, the non-human mammal is a goat. In some embodiments, the compositions comprising populations of antibodies that are highly galactosylated further comprise milk.

The populations of antibodies that are transgenically produced according to the methods disclosed herein are highly galactosylated and highly fucosylated. The art provides that high-mannose antibodies / low fucose antibodies have been transgenically produced (See *e.g.,* WO 2007/048077).

*CDC activity*

In one aspect, the compositions comprising populations of antibodies that are highly galactosylated have high complement dependent cytotoxicity (CDC) activity. In one aspect, the compositions comprising populations of antibodies that are highly galactosylated have high antibody-dependent cellular cytotoxicity (ADCC) activity. In some embodiments, the compositions comprising populations of antibodies that are highly galactosylated have high complement dependent cytotoxicity (CDC) activity and have high antibody-dependent cellular cytotoxicity (ADCC) activity.

In some embodiments, the population of antibodies that are highly galactosylated has an increased level of complement dependent cytotoxicity (CDC) activity when compared to a population of antibodies that are low galactose. In some embodiments, the population of antibodies that is highly galactosylated and the population of antibodies that are low galactose are directed to the same antigen epitope. In some embodiments, the population of antibodies that is highly galactosylated and the population of antibodies that are low galactose are encoded by the same nucleic acid.

A population of antibodies that are low galactose, as used herein, refers to a population of antibodies wherein the level of galactosylation of the antibodies in the population is less than 50 %, less than 40%, less than 30%, less than 20%, less than 10%, down to 0%.

In some embodiments, the CDC activity of a population of antibodies that is highly galactosylated is at least 1.1 times higher, at least 1.2 times higher, at least 1.3 times higher, at least 1.4 times higher, at least 1.5 times higher, at least 1.6 times higher, at least 1.7 times higher, at least 1.8 times higher, at least 1.9 times higher, at least 2 times higher, at least 3 times higher, at least 5 times higher, at least 10 times higher, up to at least 100 times higher or more when compared to a population of antibodies that are low galactose. In some embodiments, the CDC activity of a population of antibodies that is highly galactosylated is at least 1.5 times higher when compared to a population of antibodies that are low galactose. In some embodiments, the CDC activity of a population of antibodies that is highly galactosylated is at least 3 times higher when compared to a population of antibodies that are low galactose.

In some embodiments, the population of antibodies that are highly galactosylated is highly fucosylated. In some embodiments, the population of antibodies that are highly galactosylated and highly fucosylated has an increased level of complement dependent cytotoxicity (CDC) activity when compared to a population of antibodies that are low galactose and low fucose. In some embodiments, the population of antibodies that is highly galactosylated and highly fucosylated and the population of antibodies that is are low galactose and low fucose are directed to the same antigen epitope. In some embodiments, the population of antibodies that is highly galactosylated and highly fucosylated and the population of antibodies that is are low galactose and low fucose are encoded by the same nucleic acid.

A population of antibodies that are low fucose, as used herein, refers to a population of antibodies wherein the level of fucosylation of the antibodies in the population is less than 50%, less than 40%, less than 30%, less than 20%, less than 10%, down to 0%.

In some embodiments, the CDC activity of a population of antibodies that is highly galactosylated and highly fucosylated is at least 1.1 times higher, at least 1.2 times higher, at least 1.3 times higher, at least 1.4 times higher, at least 1.5 times higher, at least 1.6 times higher, at least 1.7 times higher, at least 1.8 times higher, at least 1.9 times higher, at least 2 times higher, at least 3 times higher, at least 5 times higher, at least 10 times higher, up to at least 100 times higher or more when compared to a population of antibodies that are low galactose and low fucose. In some embodiments, the CDC activity of a population of antibodies that is highly galactosylated and highly fucosylated is at least 1.5 times higher when compared to a population of antibodies that are low galactose and low fucose. In some embodiments, the CDC activity of a population of antibodies that is highly galactosylated and highly fucosylated is at least 3 times higher when compared to a population of antibodies that are low galactose and low fucose.

In some embodiments, the population of antibodies that is highly galactosylated and is produced in mammary gland epithelial cells has an increased level of complement dependent cytotoxicity (CDC) activity when compared to a population of antibodies that is not produced in mammary gland epithelial cells. In some embodiments, the population of antibodies not produced in mammary gland epithelial cells is produced in cell culture. In some embodiments, the population of antibodies produced in cell culture is Rituxan. In some embodiments, the population of antibodies that is highly galactosylated produced in mammary gland epithelial cells and the population of antibodies that is not produced in mammary gland epithelial cells may be encoded by the same nucleic acid.

In some embodiments, the CDC activity of a population of antibodies that is highly galactosylated and is produced in mammary gland epithelial cells is at least 1.1 times higher, at least 1.2 times higher, at least 1.3 times higher, at least 1.4 times higher, at least 1.5 times higher, at least 1.6 times higher, at least 1.7 times higher, at least 1.8 times higher, at least 1.9 times higher, at least 2 times higher, at least 3 times higher, at least 5 times higher, at least 10 times higher, up to at least 100 times higher or more when compared to a population of antibodies that is not produced in mammary gland epithelial cells. In some embodiments, the CDC activity of a population of antibodies that is highly galactosylated and is produced in mammary gland epithelial cells is at least 1.5 times higher when compared to a population of antibodies that is not produced in mammary gland epithelial cells. In some embodiments, the CDC activity of a population of antibodies that is highly galactosylated and is produced in mammary gland epithelial cells is at least 3 times higher when compared to a population of antibodies that is not produced in mammary gland epithelial cells.

In one aspect, the compositions of the populations of antibodies disclosed herein have a high (complement dependent cytotoxicity) CDC activity. Antibodies can act as a therapeutic through various mechanisms, one of which is through CDC activity. Some therapeutic antibodies that bind to target cellular receptors (such as CD20) can also bind proteins of the complement pathway. Binding of the complement proteins results in a complement cascade (through C1-complex activation) that eventually results in the formation of a "membrane attack complex" causing cell lysis and death of the cell to which the therapeutic antibody is bound (See *e.g.,* Reff M. E. Blood 1994, 83: 435). One example of a therapeutic antibody that is thought to exert its therapeutic effect, at least in part, through the induction of CDC activity is Rituximab (Rituxan), which can bind CD20 (which is overexpressed on certain lymphoma cells).

In some embodiments a population of antibodies that has an increased level of complement dependent cytotoxicity (CDC) activity, is a population of antibodies that induces a larger amount of cell lysis as compared to a population of antibodies that has does not have an increased level of complement dependent cytotoxicity (CDC) activity. Methods for determining the level of CDC are known in the art and are often based on determining the amount of cell lysis. Commercial kits for determining CDC activity can be purchased for instance from Genscript (Piscataway, NJ).

*ADCC activity*

In one aspect, the compositions comprising populations of antibodies that are highly galactosylated have high complement dependent cytotoxicity (CDC) activity. In one aspect, the compositions comprising populations of antibodies that are highly galactosylated have high antibody-dependent cellular cytotoxicity (ADCC) activity. In some embodiments, the compositions comprising populations of antibodies that are highly galactosylated have high complement dependent cytotoxicity (CDC) activity and have high antibody-dependent cellular cytotoxicity (ADCC) activity.

In some embodiments, the population of antibodies that are highly galactosylated has an increased level of antibody-dependent cellular cytotoxicity (ADCC) when compared to a population of antibodies that are low galactose. In some embodiments, the ADCC activity of a population of antibodies that is highly galactosylated is at least 1.1 times higher, 1.2 times higher, 1.3 times higher, 1.4 times higher, 1.5 times higher, 1.6 times higher, 1.7 times higher, 1.8 times higher, 1.9 times higher, 2 times higher, 3 times higher, 5 times higher, 10 times higher, 100 times higher or more when compared to a population of antibodies that are low galactose. In some embodiments, the ADCC activity of a population of antibodies that is highly galactosylated is at least 2 times higher when compared to a population of antibodies that are low galactose. In some embodiments, the ADCC activity of a population of antibodies that is highly galactosylated is at least 5 times higher when compared to a population of antibodies that are low galactose.

In some embodiments, the population of antibodies that are highly galactosylated and is produced in mammary gland epithelial cells has an increased level of antibody-dependent cellular cytotoxicity (ADCC) when compared to a population of antibodies that is not produced in mammary gland epithelial cells. In some embodiments, the ADCC activity of a population of antibodies that is highly galactosylated and produced in mammary gland epithelial cells is at least 1.1 times higher, 1.2 times higher, 1.3 times higher, 1.4 times higher, 1.5 times higher, 1.6 times higher, 1.7 times higher, 1.8 times higher, 1.9 times higher, 2 times higher, 3 times higher, 5 times higher, 10 times higher, 100 times higher or more when compared to a population of antibodies that is not produced in mammary gland epithelial cells. In some embodiments, the ADCC activity of a population of antibodies that is highly galactosylated and produced in mammary gland epithelial cells is at least 2 times higher when compared to a population of antibodies that is not produced in mammary gland epithelial cells. In some embodiments, the ADCC activity of a population of antibodies that is highly galactosylated and produced in mammary gland epithelial cells is at least 5 times higher when compared to a population of antibodies that is not produced in mammary gland epithelial cells.

In some embodiments, the population of antibodies that is highly galactosylated and is produced in mammary gland epithelial cells has an increased level of antibody-dependent cellular cytotoxicity (ADCC) when compared to a population of antibodies that is not produced in mammary gland epithelial cells. In some embodiments, the population of antibodies not produced in mammary gland epithelial cells is produced in cell culture. In some embodiments, the population of antibodies produced in cell culture is Rituxan.

In some embodiments, the population of antibodies that are highly galactosylated is highly fucosylated. In some embodiments, the population of antibodies that are highly galactosylated and highly fucosylated has a significant percentage of the antibody-dependent cellular cytotoxicity (ADCC) activity of a population of antibodies that are low galactose and low fucose. In some embodiments, the ADCC activity of a population of antibodies that is highly galactosylated and highly fucosylated is has least 10%, at least 20%, at least 30%, at least 40%, at least 50%, at least 60%, at least 70%, at least 80%, at least 90%, up to 100% or more, when compared to a population of antibodies that are low galactose and low fucose. In some embodiments, the ADCC activity of a population of antibodies that is highly galactosylated and highly fucosylated is at least 40% when compared to a population of antibodies that are low galactose and low fucose.

In some embodiments, the population of antibodies that are highly galactosylated and produced in mammary gland epithelial cells has a significant percentage of the antibody-dependent cellular cytotoxicity (ADCC) activity of a population of antibodies that is not produced in mammary gland epithelial cells. In some embodiments, the population of antibodies that is highly galactosylated produced in mammary gland epithelial cells and the population of antibodies that is not produced in mammary gland epithelial cells may be encoded by the same nucleic acid. In some embodiments, the population of antibodies not produced in mammary gland epithelial cells is produced in cell culture. In some embodiments, the ADCC activity of a population of antibodies that is highly galactosylated and produced in mammary gland epithelial cells is at least 10%, at least 20%, at least 30%, at least 40%, at least 50%, at least 60%, at least 70%, at least 80%, at least 90%, up to 100% or more, when compared to a population of antibodies that is not produced in mammary gland epithelial cells. In some embodiments, the ADCC activity of a population of antibodies that is highly galactosylated and produced in mammary gland epithelial cells is at least 40% when compared to a population of antibodies that is not produced in mammary gland epithelial cells.

In one aspect, the compositions of the populations of antibodies disclosed herein have a high ADCC activity. Antibodies can act as a therapeutic through various mechanisms, one of which is through ADCC activity. Therapeutic antibodies that bind to cellular receptors (such as CD20) on a target cell, and that include the Fc glycosylation site can also bind the Fc-receptor (such as CD16) resulting in the anchoring of cells expressing the Fc-receptor to the target cell. The affinity of binding of the Fc regions of antibodies generally is dependent on the nature of the glycosylation of the Fc glycosylation site. The Fc receptor is found on a number of immune cells including natural killer cells, macrophages, neutrophils, and mast cells. Binding to the Fc receptor results in the immune cells inducing cytokines (such as IL-2) and phagocytosis to kill the target cell. In some embodiments, a population of antibodies that has an increased level of antibody-dependent cellular cytotoxicity (ADCC) activity is a population of antibodies that shows increased binding to cells expressing CD16 as compared to a population of antibodies that does not have an increased level of antibody-dependent cellular cytotoxicity (ADCC) activity. In some embodiments a population of antibodies that has an increased level of antibody-dependent cellular cytotoxicity (ADCC) activity is a population of antibodies that shows increased induction of IL-2 production (*e.g.,* in natural killer cells) as compared to a population of antibodies that has does not have an increased level of antibody-dependent cellular cytotoxicity (ADCC) activity. Commercial kits for determining ADCC activity can be purchased for instance from Genscript (Piscataway, NJ) and Promega (Madison, WI).

*B-cell depletion*

In one aspect, the compositions comprising populations of antibodies that are highly galactosylated have high complement dependent cytotoxicity (CDC) activity. In one aspect, the compositions comprising populations of antibodies that are highly galactosylated have high antibody-dependent cellular cytotoxicity (ADCC) activity. In some embodiments, the compositions comprising populations of antibodies that are highly galactosylated have high complement dependent cytotoxicity (CDC) activity and have high antibody-dependent cellular cytotoxicity (ADCC) activity. In some embodiments, the compositions comprising populations of antibodies that are highly galactosylated have a strong ability to induce B cell depletion (*e.g.,* in blood sample or in a subject). In some embodiments, the antibodies of the populations of antibodies that are highly galactosylated and have a strong ability to induce B cell depletion are anti-CD20 antibodies.

*Antibodies*

In one aspect, the disclosure provides populations of highly galactosylated antibodies. In some embodiments, the populations of highly galactosylated antibodies are transgenically produced. In some embodiments, the antibodies in the population are chimeric, humanized or fully human antibodies. In some embodiments, the antibodies in the population are full-length antibodies. In some embodiments, the antibodies in the population comprise a heavy chain and a light chain.

In some embodiments, the antibody of the population of antibodies is an anti-CD20 antibody. Anti CD-20 antibodies are described in the art and include for instance, rituximab (Genentech), ofatumumab (GSK) and ocrelizumab (Genentech) (See *e.g.,* Robak et al., Biodrugs 2011, 25: 13-25). In some embodiments, the population of antibodies is a mixture of anti-CD20 antibodies.

In some embodiments, the antibody of the population of antibodies is an antibody wherein the light chain and heavy chain of the antibodies in the population are encoded by nucleic acid sequences as set forth in SEQ ID NO:1 and SEQ ID NO:2

A "population of antibodies", as used herein, refers to a batch of antibodies that are directed against the same antigen (*e.g.,* anti-CD20 antibodies). In some embodiments, the antibodies of the population of antibodies are directed against the same antigen epitope (*e.g.,* anti-CD20 antibodies that bind a particular amino acid sequence of CD20). A population of antibodies that are directed against the same epitope include a population of antibodies that have the same CDRs but have different non-CDR regions, and include a population of antibodies wherein some of the antibodies have one or more mutations that do not change the nature of binding to the antigen epitope to which the antibody is directed. In some embodiments, the antibodies of the population of antibodies are encoded by the same nucleic acid. However, it should be appreciated that even if the antibodies in a population of antibodies are encoded by the same nucleic acid, they do not need to be identical. For instance, the antibodies may have different glycosylation patterns.

In some embodiments, the antibodies of the population of antibodies are produced in a similar manner. For instance, a population of antibodies may be produced transgenically. The population may originate from one harvest or multiple harvests of transgenically produced antibody. The multiple harvests can be from the same source, *e.g.,* the same transgenic animal or may be combined from different sources, *e.g.,* a different transgenic animal.

In some embodiments, the antibodies are transgenically produced antibodies. The term "transgenically produced antibodies" as used herein refers to antibodies that are produced in a transgenic animal, *i.e.,* an animal that has in its genome the nucleic acid sequence encoding the antibody to be produced. In some embodiments, the transgenic antibody is expressed in one or more of the organs of the transgenic animal. In some embodiments, the transgenic antibody is expressed the liver. In some embodiments, the transgenic antibody is expressed in the mammary gland epithelial cells.

An "isolated antibody", as used herein, refers to an antibody which is substantially free of other antibodies having different antigenic specificities. An isolated antibody that specifically binds to an epitope, isoform or variant of an antigen may, however, have cross-reactivity to other related antigens, *e.g.,* from other species. Moreover, an isolated antibody may be substantially free of other cellular material and/or chemicals. As used herein, "specific binding" refers to antibody binding to a predetermined antigen. Typically, the antibody binds with an affinity that is at least two-fold greater than its affinity for binding to a non-specific antigen other than the predetermined antigen or a closely-related antigen. Therefore, the antibodies provided herein in some embodiments specifically bind a target antigen.

As used herein, the term "antibody" refers to a glycoprotein comprising at least two heavy (H) chains and two light (L) chains inter-connected by disulfide bonds, *i.e.,* covalent heterotetramers comprised of two identical Ig H chains and two identical L chains that are encoded by different genes. Each heavy chain is comprised of a heavy chain variable region (abbreviated herein as HCVR or $V_H$) and a heavy chain constant region. The heavy chain constant region is comprised of three domains, $C_H1$, $C_H2$ and $C_H3$. Each light chain is comprised of a light chain variable region (abbreviated herein as LCVR or $V_L$) and a light chain constant region. The light chain constant region is comprised of one domain, CL. The $V_H$ and $V_L$ regions can be further subdivided into regions of hypervariability, termed complementarity determining regions (CDR), interspersed with regions that are more conserved, termed framework regions (FR). Each $V_H$ and $V_L$ is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (*e.g.,* effector cells) and the first component (C1q) of the classical complement system. Formation of a mature functional antibody molecule can be accomplished when two proteins are expressed in stoichiometric quantities and self-assemble with the proper configuration.

The term "antibodies" also encompasses antigen-binding fragments thereof. As used herein, an "antigen-binding fragment" of an antibody refers to one or more portions of an antibody that retain the ability to specifically bind to an antigen and includes the Fc glycosylation site.

In some embodiments, the antibodies are of the isotype IgG, IgA or IgD. In further embodiments, the antibodies are selected from the group consisting of IgG1, IgG2, IgG3, IgG4, IgM, IgA1, IgA2, IgAsec, IgD, IgE or has immunoglobulin constant and/or variable domain of IgG1, IgG2, IgG3, IgG4, IgM, IgA1, IgA2, IgAsec, IgD or IgE. In preferred embodiments, the antibodies are of the type IgG1. In other embodiments, the antibodies are bispecific or multispecific antibodies. In still other embodiments, the antibodies are recombinant antibodies, polyclonal antibodies, monoclonal antibodies, humanized antibodies or chimeric antibodies, or a mixture of these. In some embodiments, the chimeric antibody is a genetically engineered fusion of parts of a non-human (*e.g.,* mouse, rat or rabbit) antibody with parts of a human antibody. The chimeric antibodies, in some embodiments, can contain approximately 33% non-human protein and 67% human protein. With specific regard to mouse chimerics, they can be developed to reduce the HAMA response elicited by murine antibodies, as they can combine the specificity of the murine antibody with the efficient human immune system interaction of a human antibody.

In some embodiments, the antibodies are chimeric or humanized antibodies. As used herein, the term "chimeric antibody" refers to an antibody that combines the murine variable or hypervariable regions with the human constant region or constant and variable framework regions. As used herein, the term "humanized antibody" refers to an antibody that retains only the antigen-binding CDRs from the parent antibody in association with human framework regions (see, Waldmann, 1991, Science 252:1657). Such chimeric or humanized antibodies retaining binding specificity of the murine antibody are expected to have reduced immunogenicity when administered *in vivo* for diagnostic, prophylactic or therapeutic applications as described herein. Humanization (also called Reshaping or CDR-grafting) is an established technique for reducing the immunogenicity of monoclonal antibodies from xenogeneic sources, such as mice. Humanized antibodies can be generated through standard molecular biology techniques. In some embodiments, this comprises grafting of the rodent complementarity-determining regions (CDRs) into a human framework. However, this technique is mostly an iterative process and a number of elements come into play when designing a humanized antibody: the length of the CDRs, the human frameworks and the substitution of residues from the rodent mAb into the human framework regions (backmutations).

Therapeutic mouse mAbs are at times not ideal for human use because the HAMA (human anti-mouse antibodies) response neutralizes the antibody, and clears it quickly from the circulation and, in the worst case, induces serious allergic hypersensitivity. Several strategies have been developed to replace most of the murine Ig sequences with human sequences, resulting in fewer side effects while retaining efficacy. One strategy for developing a human therapeutic mAb is to replace the murine heavy chain (H) and light chain (L) constant regions ($C_H$ and $C_L$, respectively), or generically non-human chains, with human regions so that the resulting chimeric antibody is comprised mostly of human IgG protein sequence except for the antigen-binding domains that would remain non-human. This strategy was used for the development of Rituxan® (Rituximab anti-human CD20, Genentech), the first monoclonal antibody approved in the U.S., used to treat non-Hodgkin lymphoma. By some estimates, providing therapeutic mAbs with human $C_H$ and $C_L$ sequences should eliminate approximately 90% of the immunogenicity of murine antibody proteins.

In certain embodiments, the antibodies are human antibodies. The term "human antibody", as used herein, is intended to include antibodies having variable and constant regions derived from human germline immunoglobulin sequences. The human antibodies described herein may include amino acid residues not encoded by human germline immunoglobulin sequences (e.g., mutations introduced by random or site-specific mutagenesis *in vitro* or by somatic mutation *in vivo)*. However, the term "human antibody", as used herein, is not intended to include antibodies in which CDR sequences derived from the germline of another mammalian species, such as a mouse have been grafted onto human framework sequences (referred to herein as "humanized antibodies"). Human antibodies are generated using transgenic mice carrying parts of the human immune system rather than the mouse system.

Fully human monoclonal antibodies also can be prepared by immunizing mice transgenic for large portions of human immunoglobulin heavy and light chain loci. See, *e.g.,* U.S. patents 5,591,669, 5,598,369, 5,545,806, 5,545,807, 6,150,584, and references cited therein, the contents of which are incorporated herein by reference. These animals have been genetically modified such that there is a functional deletion in the production of endogenous (*e.g.,* murine) antibodies. The animals are further modified to contain all or a portion of the human germ-line immunoglobulin gene locus such that immunization of these animals results in the production of fully human antibodies to the antigen of interest. Following immunization of these mice (*e.g.,* XenoMouse (Abgenix), HuMAb mice (Medarex/GenPharm)), monoclonal antibodies are prepared according to standard hybridoma technology. These monoclonal antibodies have human immunoglobulin amino acid sequences and therefore will not provoke human anti-mouse antibody (HAMA) responses when administered to humans. The human antibodies, like any of the antibodies provided herein can be monoclonal antibodies, polyclonal antibodies or a mixture of monoclonal and polyclonal antibodies.

*Sources of highly galactosylated antibodies*

In one aspect, it is described a population of antibodies that is highly galactosylated. In some embodiments, the population of highly galactosylated antibodies is produced transgenically.

In some embodiments, mammalian mammary epithelial cells have been engineered to express the antibody in the milk of a transgenic animal, such as a mouse or goat. The expression of this gene is, for example, under the control of the goat β-casein regulatory elements. The transgenic animals can be generated by co-transfecting separate constructs containing the H and L chains, or one construct containing both chains. In certain embodiments, both transgenes integrate into the same chromosomal site so that the genes are transmitted together to progeny and protein expression is jointly regulated. In some embodiments, the expression is optimized for individual mammary duct epithelial cells that produce milk proteins.

*Constructs for the generation of transgenic animals*

In some embodiments, to produce primary cell lines containing a construct (*e.g.,* encoding an anti-CD20 antibody) for use in producing transgenic goats by nuclear transfer, the heavy and light chain constructs can be transfected into primary goat skin epithelial cells, which are clonally expanded and fully characterized to assess transgene copy number, transgene structural integrity and chromosomal integration site. As used herein, "nuclear transfer" refers to a method of cloning wherein the nucleus from a donor cell is transplanted into an enucleated oocyte.

Coding sequences for proteins of interest (*e.g.,* an antibody) can be obtained by screening libraries of genomic material or reverse-translated messenger RNA derived from the animal of choice (such as cattle or mice), obtained from sequence databases such as NCBI, Genbank, or by obtaining the sequences of antibodies, etc. The sequences can be cloned into an appropriate plasmid vector and amplified in a suitable host organism, like *E. coli.* After amplification of the vector, the DNA construct can be excised, purified from the remains of the vector and introduced into expression vectors that can be used to produce transgenic animals. The transgenic animals will have the desired transgenic protein integrated into their genome.

After amplification of the vector, the DNA construct can be excised with the appropriate 5' and 3' control sequences, purified away from the remains of the vector and used to produce transgenic animals that have integrated into their genome the desired non-glycosylated related transgenic protein. Conversely, with some vectors, such as yeast artificial chromosomes (YACs), it is not necessary to remove the assembled construct from the vector; in such cases the amplified vector may be used directly to make transgenic animals. The coding sequence can be operatively linked to a control sequence, which enables the coding sequence to be expressed in the milk of a transgenic non-human mammal.

A DNA sequence which is suitable for directing production to the milk of transgenic animals can carry a 5'-promoter region derived from a naturally-derived milk protein. This promoter is consequently under the control of hormonal and tissue-specific factors and is most active in lactating mammary tissue. In some embodiments, the promoter is a caprine beta casein promoter. The promoter can be operably linked to a DNA sequence directing the production of a protein leader sequence, which directs the secretion of the transgenic protein across the mammary epithelium into the milk. In some embodiments a 3'-sequence, which can be derived from a naturally secreted milk protein, can be added to improve stability of mRNA.

As used herein, a "leader sequence" or "signal sequence" is a nucleic acid sequence that encodes a protein secretory signal, and, when operably linked to a downstream nucleic acid molecule encoding a transgenic protein directs secretion. The leader sequence may be the native human leader sequence, an artificially-derived leader, or may obtained from the same gene as the promoter used to direct transcription of the transgene coding sequence, or from another protein that is normally secreted from a cell, such as a mammalian mammary epithelial cell.

In some embodiments, the promoters are milk-specific promoters. As used herein, a "milk-specific promoter" is a promoter that naturally directs expression of a gene in a cell that secretes a protein into milk (*e.g.,* a mammary epithelial cell) and includes, for example, the casein promoters, *e.g.,* $\alpha$-casein promoter (*e.g.,* alpha S-1 casein promoter and alpha S2-casein promoter), $\beta$-casein promoter (*e.g.,* the goat beta casein gene promoter (DiTullio, BIOTECHNOLOGY 10:74-77, 1992), $\gamma$-casein promoter, $\kappa$-casein promoter, whey acidic protein (WAP) promoter (Gordon et al., BIOTECHNOLOGY 5: 1183-1187, 1987), $\beta$-lactoglobulin promoter (Clark et al., BIOTECHNOLOGY 7: 487-492, 1989) and $\alpha$-lactalbumin promoter (Soulier et al., FEBS LETTS. 297:13, 1992). Also included in this definition are promoters that are specifically activated in mammary tissue, such as, for example, the long terminal repeat (LTR) promoter of the mouse mammary tumor virus (MMTV).

As used herein, a coding sequence and regulatory sequences are said to be "operably joined" when they are covalently linked in such a way as to place the expression or transcription of the coding sequence under the influence or control of the regulatory sequences. In order for the coding sequences to be translated into a functional protein the coding sequences are operably joined to regulatory sequences. Two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region is operably joined to a coding sequence if the promoter region were capable of effecting transcription of that DNA sequence such that the resulting transcript might be translated into the desired protein or polypeptide.

As used herein, a "vector" may be any of a number of nucleic acids into which a desired sequence may be inserted by restriction and ligation for transport between different genetic environments or for expression in a host cell. Vectors are typically composed of DNA although RNA vectors are also available. Vectors include, but are not limited to, plasmids and phagemids. A cloning vector is one which is able to replicate in a host cell, and which is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium, or just a single time per host as the host reproduces by mitosis. In the case of phage, replication may occur actively during a lytic phase or passively during a lysogenic phase. An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells, which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (*e.g.,* $\beta$-galactosidase or alkaline phosphatase), and genes which visibly affect the phenotype of transformed or transfected cells, hosts, colonies or plaques. Preferred vectors are those capable of autonomous replication and expression of the structural gene products present in the DNA segments to which they are operably joined.

*Transgenic animals*

In one aspect, the disclosure provides mammary gland epithelial cells that express any of the population of antibodies provided herein. In some embodiments, the disclosure provides a transgenic non-human mammal comprising the mammary gland epithelial cells mammary gland epithelial cells that express any of the population of antibodies provided herein.

In one aspect, the disclosure provides a method for the production of a transgenic antibody, and variants and fragments thereof, the process comprising expressing in the milk of a transgenic non-human mammal a transgenic antibody encoded by a nucleic acid construct. In some embodiments, the method for producing the antibodies described herein comprises:

(a) transfecting non-human mammalian cells with a transgene DNA construct encoding a desired transgenic antibody;
(b) selecting cells in which said transgene DNA construct has been inserted into the genome of the cells; and
(c) performing a first nuclear transfer procedure to generate a non-human transgenic mammal heterozygous for the desired transgenic antibody and that can express it in its milk.

In one aspect, the disclosure provides a method of

(a) providing a non-human transgenic mammal engineered to express an antibody,
(b) expressing the antibody in the milk of the non-human transgenic mammal; and
(c) isolating the antibodies expressed in the milk.

Such methods can further comprise steps for inducing lactation as well as steps for determining the CDC activity and/or the ADCC activity of the antibodies obtained. Such methods can further comprise steps for determining the amount or level of galactosylation and or fucosylation of the antibodies obtained. The methods can also further comprise additional isolation and/or purification steps. The methods can also comprise steps for comparing the CDC activity and/or the ADCC activity of the antibodies obtained with antibodies not produced in mammary gland epithelial cells (*e.g.,* produced in cell culture).

Transgenic animals, capable of recombinant antibody expression, can also be generated according to methods known in the art (See *e.g.,* U.S. Patent No. 5,945,577). Animals suitable for transgenic expression, include, but are not limited to goat, sheep, bison, camel, cow, pig, rabbit, buffalo, horse, rat, mouse or llama. Suitable animals also include bovine, caprine, ovine and porcine, which relate to various species of cows, goats, sheep and pigs (or swine), respectively. Suitable animals also include ungulates. As used herein, "ungulate" is of or relating to a hoofed typically herbivorous quadruped mammal, including, without limitation, sheep, swine, goats, cattle and horses. In one embodiment, the animals are generated by co-transfecting primary cells with separate constructs containing the heavy and light chains. These cells are then used for nuclear transfer. Alternatively, if microinjection is used to generate the transgenic animals, the constructs may be-injected.

Cloning will result in a multiplicity of transgenic animals - each capable of producing an antibody or other gene construct of interest. The production methods include the use of the cloned animals and the offspring of those animals. In some embodiments, the cloned animals are caprines, bovines or mice. Cloning also encompasses the nuclear transfer of fetuses, nuclear transfer, tissue and organ transplantation and the creation of chimeric offspring.

One step of the cloning process comprises transferring the genome of a cell that contains the transgene encoding the antibody into an enucleated oocyte. As used herein, "transgene" refers to any piece of a nucleic acid molecule that is inserted by artifice into a cell, or an ancestor thereof, and becomes part of the genome of an animal which develops from that cell. Such a transgene may include a gene which is partly or entirely exogenous (*i.e.,* foreign) to the transgenic animal, or may represent a gene having identity to an endogenous gene of the animal.

Suitable mammalian sources for oocytes include goats, sheep, cows, pigs, rabbits, guinea pigs, mice, hamsters, rats, non-human primates, etc. Preferably, oocytes are obtained from ungulates, and most preferably goats or cattle. Methods for isolation of oocytes are well known in the art. Essentially, the process comprises isolating oocytes from the ovaries or reproductive tract of a mammal, *e.g.,* a goat. A readily available source of ungulate oocytes is from hormonally-induced female animals. For the successful use of techniques such as genetic engineering, nuclear transfer and cloning, oocytes may preferably be matured *in vivo* before these cells may be used as recipient cells for nuclear transfer, and before they were fertilized by the sperm cell to develop into an embryo. Metaphase II stage oocytes, which have been matured *in vivo,* have been successfully used in nuclear transfer techniques. Essentially, mature metaphase II oocytes are collected surgically from either non-super ovulated or super ovulated animals several hours past the onset of estrus or past the injection of human chorionic gonadotropin (hCG) or similar hormone.

One of the tools used to predict the quantity and quality of the recombinant protein expressed in the mammary gland is through the induction of lactation (Ebert KM, 1994). Induced lactation allows for the expression and analysis of protein from the early stage of transgenic production rather than from the first natural lactation resulting from pregnancy, which is at least a year later. Induction of lactation can be done either hormonally or manually.

In some embodiments, the compositions of highly galactosylated antibodies provided herein further comprise milk. In some embodiments, the methods provides herein includes a step of isolating the population of antibodies from the milk of a transgenic animal. Methods for isolating antibodies from the milk of transgenic animal are known in the art and are described for instance in Pollock et al., Journal of Immunological Methods, Volume 231, Issues 1-2, 10 December 1999, Pages 147-157

*Treatment of diseases*

In one aspect, the disclosure provides methods for administering any one of the compositions described herein to a subject in need thereof, such as a subject affected by a disease, by a trauma or by a poisoning.

In some embodiments, the subject has cancer. In some embodiments, the cancer is B-cell lymphoma.

"Cancer" as used herein refers to an uncontrolled growth of cells which interferes with the normal functioning of the bodily organs and systems. Cancers which migrate from their original location and seed vital organs can eventually lead to the death of the subject through the functional deterioration of the affected organs.

Cancer, as used herein, includes the following types of cancers, B-cell lymphoma, Hodgkin's lymphoma, non-Hodgkin's lymphoma, mycosis fungoides/Sezary syndrome, histiocytosis X, chronic lymphocytic leukaemia, hairy cell leukaemia, multiple myeloma, Waldenstrom's macroglobulinaemia, cryoglobulinaemi, heavy chain disease, breast cancer, biliary tract cancer; bladder cancer; brain cancer including glioblastomas and medulloblastomas; cervical cancer; choriocarcinoma; colon cancer; endometrial cancer; esophageal cancer; gastric cancer; leukemia; hematological neoplasms including acute lymphocytic and myelogenous leukemia; T-cell acute lymphoblastic leukemia/lymphoma; hairy cell leukemia; chromic myelogenous leukemia, multiple myeloma; AIDS-associated leukemias and adult T-cell leukemia lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Kaposi's sarcoma, basocellular cancer, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor. Other cancers will be known to one of ordinary skill in the art and include mastocytoma, thymoma, plasmacytoma and glioma.

The methods of treatment are also directed to hemopoietic cancers, such as leukemia, which are able to outcompete the normal hemopoietic compartments in a subject, thereby leading to hemopoietic failure (in the form of anemia, thrombocytopenia and neutropenia) ultimately causing death.

The methods of treatment are also directed to the suppression of metastasis. A metastasis is a region of cancer cells, distinct from the primary tumor location resulting from the dissemination of cancer cells from the primary tumor to other parts of the body. At the time of diagnosis of the primary tumor mass, the subject may be monitored for the presence of metastases. Metastases are most often detected through the sole or combined use of magnetic resonance imaging (MRI) scans, computed tomography (CT) scans, blood and platelet counts, liver function studies, chest X-rays and bone scans in addition to the monitoring of specific symptoms.

*Treatment of immune disorders*

In one aspect, the disclosure provides methods for administering any one of the compositions described herein to a subject in need thereof. In some embodiments, the subject has an immune disorder.

The compositions described herein are also useful for treating immune disorders, which include but are not limited to adult respiratory distress syndrome, arteriosclerosis, asthma, atherosclerosis, cholecystitis, cirrhosis, Crohn's disease, diabetes mellitus, emphysema, hypereosinophilia, inflammation, irritable bowel syndrome, multiple sclerosis, myasthenia gravis, myocardial or pericardial inflammation, osteoarthritis, osteoporosis, pancreatitis, rheumatoid arthritis, scleroderma, colitis, systemic lupus erythematosus, lupus nephritis, diabetes mellitus, inflammatory bowel disease, celiac disease, an autoimmune thyroid disease, Addison's disease, Sjogren's syndrome, Sydenham's chorea, Takayasu's arteritis, Wegener's granulomatosis, autoimmune gastritis, autoimmune hepatitis, cutaneous autoimmune diseases, autoimmune dilated cardiomyopathy, multiple sclerosis, myocarditis, myasthenia gravis, pernicious anemia, polymyalgia, psoriasis, rapidly progressive glomerulonephritis, rheumatoid arthritis, ulcerative colitis, vasculitis, autoimmune diseases of the muscle, autoimmune diseases of the testis, autoimmune diseases of the ovary and autoimmune diseases of the eye.

*Other therapeutic agents*

In one aspect, the antibody compositions provided herein are administered with other therapeutic agents. The antibody compositions and other therapeutic agent may be administered simultaneously or sequentially. When the other therapeutic agents are administered simultaneously they can be administered in the same or separate formulations, but are administered at the same time. The other therapeutic agents are administered sequentially with one another and with the antibodies, when the administration of the other therapeutic agents and the antibodies is temporally separated. The separation in time between the administration of these compounds may be a matter of minutes or it may be longer.

Other therapeutic agents include, for example, but are not limited to anti cancer therapies. Anti-cancer therapies include cancer medicaments, radiation and surgical procedures. As used herein, a "cancer medicament" refers to an agent which is administered to a subject for the purpose of treating a cancer.

As used herein, "treating cancer" includes preventing the development of a cancer, reducing the symptoms of cancer, and/or inhibiting the growth of an established cancer. In other aspects, the cancer medicament is administered to a subject at risk of developing a cancer for the purpose of reducing the risk of developing the cancer. Various types of medicaments for the treatment of cancer are described herein. For the purpose of this specification, cancer medicaments are classified as chemotherapeutic agents, immunotherapeutic agents, cancer vaccines, hormone therapy and biological response modifiers.

The chemotherapeutic agent may be selected from the group consisting of methotrexate, vincristine, adriamycin, cisplatin, non-sugar containing chloroethylnitrosoureas, 5-fluorouracil, mitomycin C, bleomycin, doxorubicin, dacarbazine, taxol, fragyline, Meglamine GLA, valrubicin, carmustaine and poliferposan, MMI270, BAY 12-9566, RAS famesyl transferase inhibitor, famesyl transferase inhibitor, MMP, MTA/LY231514, LY264618/Lometexol, Glamolec, CI-994, TNP-470, Hycamtin/Topotecan, PKC412, Valspodar/PSC833, Novantrone/Mitroxantrone, Metaret/Suramin, Batimastat, E7070, BCH-4556, CS-682, 9-AC, AG3340, AG3433, Incel/VX-710, VX-853, ZD0101, ISI641, ODN 698, TA 2516/Marmistat, BB2516/Marmistat, CDP 845, D2163, PD183805, DX8951f, Lemonal DP 2202, FK 317, Picibanil/OK-432, AD 32/Valrubicin, Metastron/strontium derivative, Temodal/Temozolomide, Evacet/liposomal doxorubicin, Yewtaxan/Paclitaxel, Taxol/Paclitaxel, Xeload/Capecitabine, Furtulon/Doxifluridine, Cyclopax/oral paclitaxel, Oral Taxoid, SPU-077/Cisplatin, HMR 1275/Flavopiridol, CP-358 (774)/EGFR, CP-609 (754)/RAS oncogene inhibitor, BMS-182751/oral platinum, UFT(Tegafur/Uracil), Ergamisol/Levamisole, Eniluracil/776C85/5FU enhancer, Campto/Levamisole, Camptosar/Irinotecan, Tumodex/Ralitrexed, Leustatin/Cladribine, Paxex/Paclitaxel, Doxil/liposomal doxorubicin, Caelyx/liposomal doxorubicin, Fludara/Fludarabine, Pharmarubicin/Epirubicin, DepoCyt, ZD1839, LU 79553/Bis-Naphtalimide, LU 103793/Dolastain, Caetyx/liposomal doxorubicin, Gemzar/Gemcitabine, ZD 0473/Anormed, YM 116, Iodine seeds, CDK4 and CDK2 inhibitors, PARP inhibitors, D4809/Dexifosamide, Ifes/Mesnex/Ifosamide, Vumon/Teniposide, Paraplatin/Carboplatin, Plantinol/cisplatin, Vepeside/Etoposide, ZD 9331, Taxotere/Docetaxel, prodrug of guanine arabinoside, Taxane Analog, nitrosoureas, alkylating agents such as melphelan and cyclophosphamide, Aminoglutethimide, Asparaginase, Busulfan, Carboplatin, Chlorombucil, Cytarabine HCI, Dactinomycin, Daunorubicin HCI, Estramustine phosphate sodium, Etoposide (VP16-213), Floxuridine, Fluorouracil (5-FU), Flutamide, Hydroxyurea (hydroxycarbamide), Ifosfamide, Interferon Alfa-2a, Alfa-2b, Leuprolide acetate (LHRH-releasing factor analogue), Lomustine (CCNU), Mechlorethamine HCI (nitrogen mustard), Mercaptopurine, Mesna, Mitotane (o.p'-DDD), Mitoxantrone HCI, Octreotide, Plicamycin, Procarbazine HCI, Streptozocin, Tamoxifen citrate, Thioguanine, Thiotepa, Vinblastine sulfate, Amsacrine (m-AMSA), Azacitidine, Erthropoietin, Hexamethylmelamine (HMM), Interleukin 2, Mitoguazone (methyl-GAG; methyl glyoxal bis-guanylhydrazone; MGBG), Pentostatin (2'deoxycoformycin), Semustine (methyl-CCNU), Teniposide (VM-26) and Vindesine sulfate, but it is not so limited.

The cancer vaccine may be selected from the group consisting of EGF, anti-idiotypic cancer vaccines, Gp75 antigen, GMK melanoma vaccine, MGV ganglioside conjugate vaccine, Her2/neu, Ovarex, M-Vax, O-Vax, L-Vax, STn-KHL theratope, BLP25 (MUC-1), liposomal idiotypic vaccine, Melacine, peptide antigen vaccines, toxin/antigen vaccines, MVA-based vaccine, PACIS, BCG vaccine, TA-HPV, TA-CIN, DISC-virus and ImmuCyst/TheraCys, but it is not so limited.

In some embodiments, the other therapeutic agent is an immunotherapeutic agent. Immunotherapeutic agents include but are not limited to Ributaxin, Herceptin, Quadramet, Panorex, IDEC-Y2B8, BEC2, C225, Oncolym, SMART M195, ATRAGEN, Ovarex, Bexxar, LDP-03, ior t6, MDX-210, MDX-11, MDX-22, OV103, 3622W94, anti-VEGF, Zenapax, MDX-220, MDX-447, MELIMMUNE-2, MELIMMUNE-1, CEACIDE, Pretarget, NovoMAb-G2, TNT, Gliomab-H, GNI-250, EMD-72000, LymphoCide, CMA 676, Monopharm-C, 4B5, ior egf.r3, ior c5, BABS, anti-FLK-2, MDX-260, ANA Ab, SMART 1D10 Ab, SMART ABL 364 Ab and ImmuRAIT-CEA.

*Pharmaceutical compositions and methods of treatment*

In one aspect, the disclosure provides compositions comprising any of the highly-galactosylated antibodies described herein and a pharmaceutically acceptable carrier.

In one aspect, the disclosure provides methods for administering any one of the compositions described herein to a subject in need thereof. In some embodiments, the subject has cancer. In some embodiments, the cancer is B-cell lymphoma. In some embodiments, the subject has an immune disorder.

A "subject" shall mean a human or vertebrate mammal including but not limited to a dog, cat, horse, cow, pig, sheep, goat, or primate, *e.g.,* monkey.

The compositions provided herein are useful in effective amounts. The term effective amount refers to the amount necessary or sufficient to realize a desired biologic effect. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial toxicity and yet is effective to treat the particular subject. The effective amount for any particular application can vary depending on such factors as the disease or condition being treated, the particular composition being administered, the size of the subject, or the severity of the disease or condition. One of ordinary skill in the art can empirically determine the effective amount of a particular composition without necessitating undue experimentation. It is preferred generally that a maximum dose be used, that is, the highest safe dose according to sound medical judgment. Multiple doses per day may be contemplated to achieve appropriate systemic levels of compounds. Appropriate system levels can be determined by, for example, measurement of the patient's peak or sustained plasma level of the drug. "Dose" and "dosage" are used interchangeably herein.

The term "treating", "treat" or "treatment" as used herein includes preventative (*e.g.,* prophylactic) and palliative treatment.

Determining a therapeutically effective amount specifically depends on such factors as toxicity and efficacy of the medicament. Toxicity may be determined using methods well known in the art. Efficacy may be determined utilizing the same guidance. A pharmaceutically effective amount, therefore, is an amount that is deemed by the clinician to be toxicologically tolerable, yet efficacious. Efficacy, for example, can be measured by the induction or substantial induction of T-lymphocyte cytotoxicity at the targeted tissue or a decrease in mass of the targeted tissue. According to a preferred embodiment suitable dosages are expected to be from about 1 mg/kg to 10 mg/kg.

According to embodiments that involve administering to a subject in need of treatment a therapeutically effective amount of the antibody compositions as provided herein, "therapeutically effective" denotes the amount of composition needed to inhibit or reverse a disease condition (*e.g.,* reduce or inhibit cancer growth). Some methods contemplate combination therapy with known cancer medicaments or therapies, for example, chemotherapy (preferably using compounds of the sort listed herein) or radiation. The patient may be a human or non-human animal. A patient typically is in need of treatment when suffering from a cancer characterized by increased levels of receptors that promote cancer maintenance or proliferation.

Generally, daily oral doses of active compounds will be from about 0.01 milligrams/kg per day to 1000 milligrams/kg per day. It is expected that oral doses in the range of 0.5 to 50 milligrams/kg, in one or several administrations per day, will yield the desired results. Dosage may be adjusted appropriately to achieve desired drug levels, local or systemic, depending upon the mode of administration. For example, it is expected that intravenous administration would be from an order to several orders of magnitude lower dose per day. In the event that the response in a subject is insufficient at such doses, even higher doses (or effective higher doses by a different, more localized delivery route) may be employed to the extent that patient tolerance permits. Multiple doses per day are contemplated in some embodiments to achieve appropriate systemic levels of antibodies.

In some embodiments, the compositions provided are employed for *in vivo* applications. Depending on the intended mode of administration *in vivo* the compositions used may be in the dosage form of solid, semi-solid or liquid such as, *e.g.,* tablets, pills, powders, capsules, gels, ointments, liquids, suspensions, or the like. Preferably, the compositions are administered in unit dosage forms suitable for single administration of precise dosage amounts. The compositions may also include, depending on the formulation desired, pharmaceutically acceptable carriers or diluents, which are defined as aqueous-based vehicles commonly used to formulate pharmaceutical compositions for animal or human administration. The diluent is selected so as not to affect the biological activity of the human recombinant protein of interest. Examples of such diluents are distilled water, physiological saline, Ringer's solution, dextrose solution, and Hank's solution. The same diluents may be used to reconstitute lyophilized a human recombinant protein of interest. In addition, the pharmaceutical composition may also include other medicinal agents, pharmaceutical agents, carriers, adjuvants, nontoxic, non-therapeutic, non-immunogenic stabilizers, etc. Effective amounts of such diluent or carrier are amounts which are effective to obtain a pharmaceutically acceptable formulation in terms of solubility of components, biological activity, etc. In some embodiments, the compositions provided herein are sterile.

The compositions herein may be administered to human patients via oral, parenteral or topical administrations and otherwise systemic forms for anti-melanoma, anti-lymphoma, anti-leukemia and anti-breast cancer treatment. The compositions described herein can also be utilized therapeutically for a range of autoimmune disorders, such as rheumatoid arthritis, systemic lupis, multiple sclerosis, etc.

Administration during *in vivo* treatment may be by any number of routes, including parenteral and oral, but preferably parenteral. Intracapsular, intravenous, intrathecal, and intraperitoneal routes of administration may be employed, generally intravenous is preferred. The skilled artisan recognizes that the route of administration varies depending on the disorder to be treated.

For use in therapy, an effective amount of the compositions can be administered to a subject by any mode that delivers the composition to the desired surface. Administering the pharmaceutical composition described herein may be accomplished by any means known to the skilled artisan. Preferred routes of administration include but are not limited to oral, parenteral, intramuscular, intranasal, sublingual, intratracheal, inhalation, ocular, vaginal, and rectal.

The compositions, when it is desirable to deliver them systemically, may be formulated for parenteral administration by injection, *e.g.,* by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the compositions may be in powder form for constitution with a suitable vehicle, *e.g.,* sterile pyrogen-free water, before use.

For oral administration, the pharmaceutical compositions may take the form of, for example, tablets or capsules prepared by conventional means with pharmaceutically acceptable excipients such as binding agents (*e.g.,* pregelatinised maize starch, polyvinylpyrrolidone or hydroxypropyl methylcellulose); fillers (*e.g.,* lactose, microcrystalline cellulose or calcium hydrogen phosphate); lubricants (*e.g.,* magnesium stearate, talc or silica); disintegrants (*e.g.,* potato starch or sodium starch glycolate); or wetting agents (*e.g.,* sodium lauryl sulphate). The tablets may be coated by methods well known in the art. Liquid preparations for oral administration may take the form of, for example, solutions, syrups or suspensions, or they maybe presented as a dry product for constitution with water or other suitable vehicle before use. Such liquid preparations may be prepared by conventional means with pharmaceutically acceptable additives such as suspending agents (*e.g.,* sorbitol syrup, cellulose derivatives or hydrogenated edible fats); emulsifying agents (*e.g.,* lecithin or acacia); non-aqueous vehicles (*e.g.,* almond oil, oily esters, ethyl alcohol or fractionated vegetable oils); and preservatives (*e.g.,* methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the composition may take the form of tablets or lozenges formulated in conventional manner.

For oral administration, for example, the compositions can be formulated readily by combining the active antibodies with pharmaceutically acceptable carriers well known in the art. Such carriers enable the compounds described herein to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl- cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers, *e.g.,* EDTA for neutralizing internal acid conditions or may be administered without any carriers.

Also specifically contemplated are oral dosage forms of the compositions. The component or components of the compositions may be chemically modified so that oral delivery of the antibody compositions is efficacious. Generally, the chemical modification contemplated is the attachment of at least one moiety to the antibodies, where said moiety permits (a) inhibition of proteolysis; and (b) uptake into the blood stream from the stomach or intestine. Also desired is the increase in overall stability of the antibodies and increase in circulation time in the body. Examples of such moieties include: polyethylene glycol, copolymers of ethylene glycol and propylene glycol, carboxymethyl cellulose, dextran, polyvinyl alcohol, polyvinyl pyrrolidone and polyproline. Abuchowski and Davis, 1981, "Soluble Polymer-Enzyme Adducts" In: Enzymes as Drugs, Hocenberg and Roberts, eds., Wiley-Interscience, New York, NY, pp. 367-383; Newmark, et al., 1982, J. Appl. Biochem. 4:185-189. Other polymers that could be used are poly-1,3-dioxolane and poly-1,3,6-tioxocane. Preferred for pharmaceutical usage, as indicated above, are polyethylene glycol moieties.

For the compositions the location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the antibody or by release of the biologically active material beyond the stomach environment, such as in the intestine.

To ensure full gastric resistance a coating impermeable to at least pH 5.0 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and Shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry therapeutic i.e. powder; for liquid forms, a soft gelatin shell may be used. The shell material of cachets could be thick starch or other edible paper. For pills, lozenges, molded tablets or tablet triturates, moist massing techniques can be used.

The composition can be included in the formulation as fine multi-particulates in the form of granules or pellets of particle size about 1 mm. The formulation of the material for capsule administration could also be as a powder, lightly compressed plugs or even as tablets. The therapeutic could be prepared by compression.

Colorants and flavoring agents may all be included. For example, the compositions may be formulated (such as by liposome or microsphere encapsulation) and then further contained within an edible product, such as a refrigerated beverage containing colorants and flavoring agents.

One may dilute or increase the volume of the therapeutic with an inert material. These diluents could include carbohydrates, especially mannitol, a-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the composition into a solid dosage form. Materials used as disintegrates include but are not limited to starch, including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the composition together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An anti-frictional agent may be included in the formulation of the composition to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the composition into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. The list of potential non-ionic detergents that could be included in the formulation as surfactants are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation either alone or as a mixture in different ratios.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the composition may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

For administration by inhalation, the composition may be conveniently delivered in the form of an aerosol spray presentation from pressurized packs or a nebulizer, with the use of a suitable propellant, *e.g.,* dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.* gelatin for use in an inhaler or insufflator may be formulated containing a powder mix of the compound and a suitable powder base such as lactose or starch.

Also contemplated herein is pulmonary delivery. The compositions can be delivered to the lungs of a mammal while inhaling and traverses across the lung epithelial lining to the blood stream. Other reports of inhaled molecules include Adjei et al., 1990, Pharmaceutical Research, 7:565-569; Adjei et al., 1990, International Journal of Pharmaceutics, 63:135-144 (leuprolide acetate); Braquet et al., 1989, Journal of Cardiovascular Pharmacology, 13(suppl. 5):143-146 (endothelin-1); Hubbard et al., 1989, Annals of Internal Medicine, Vol. III, pp. 206-212 (a1- antitrypsin); Smith et al., 1989, J. Clin. Invest. 84:1145-1146 (a-1-proteinase); Oswein et al., 1990, "Aerosolization of Proteins", Proceedings of Symposium on Respiratory Drug Delivery II, Keystone, Colorado, March, (recombinant human growth hormone); Debs et al., 1988, J. Immunol. 140:3482-3488 (interferon-g and tumor necrosis factor alpha) and Platz et al., U.S. Patent No. 5,284,656 (granulocyte colony stimulating factor). A method and composition for pulmonary delivery of drugs for systemic effect is described in U.S. Patent No. 5,451,569, issued September 19, 1995 to Wong et al.

Contemplated for use according to the present disclosure are a wide range of mechanical devices designed for pulmonary delivery of therapeutic products, including but not limited to nebulizers, metered dose inhalers, and powder inhalers, all of which are familiar to those skilled in the art.

Some specific examples of commercially available devices suitable for the delivery of the compositions are the Ultravent nebulizer, manufactured by Mallinckrodt, Inc., St. Louis, Missouri; the Acorn II nebulizer, manufactured by Marquest Medical Products, Englewood, Colorado; the Ventolin metered dose inhaler, manufactured by Glaxo Inc., Research Triangle Park, North Carolina; and the Spinhaler powder inhaler, manufactured by Fisons Corp., Bedford, Massachusetts.

All such devices require the use of formulations suitable for dispensing. Typically, each formulation is specific to the type of device employed and may involve the use of an appropriate propellant material, in addition to the usual diluents, adjuvants and/or carriers useful in therapy. Also, the use of liposomes, microcapsules or microspheres, inclusion complexes, or other types of carriers is contemplated. Chemically modified antibodies may also be prepared in different formulations depending on the type of chemical modification or the type of device employed.

Formulations suitable for use with a nebulizer, either jet or ultrasonic, will typically comprise the therapeutic dissolved in water at a concentration of about 0.1 to 25 mg of biologically active therapeutic per mL of solution. The formulation may also include a buffer and a simple sugar (*e.g.,* for antibody stabilization and regulation of osmotic pressure). The nebulizer formulation may also contain a surfactant, to reduce or prevent surface induced aggregation of the therapeutic caused by atomization of the solution in forming the aerosol.

Formulations for use with a metered-dose inhaler device will generally comprise a finely divided powder containing the therapeutic suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing the therapeutic and may also include a bulking agent, such as lactose, sorbitol, sucrose, or mannitol in amounts which facilitate dispersal of the powder from the device, *e.g.,* 50 to 90% by weight of the formulation. The therapeutic can be prepared in particulate form with an average particle size of less than 10 mm (or microns), most preferably 0.5 to 5 mm, for most effective delivery to the distal lung.

Nasal delivery of a pharmaceutical composition described herein is also contemplated. Nasal delivery allows the passage of a pharmaceutical composition described herein to the blood stream directly after administering the therapeutic product to the nose, without the necessity for deposition of the product in the lung. Formulations for nasal delivery include those with dextran or cyclodextran.

For nasal administration, a useful device is a small, hard bottle to which a metered dose sprayer is attached. In one embodiment, the metered dose is delivered by drawing the pharmaceutical composition described herein solution into a chamber of defined volume, which chamber has an aperture dimensioned to aerosolize and aerosol formulation by forming a spray when a liquid in the chamber is compressed. The chamber is compressed to administer the pharmaceutical composition described herein. In a specific embodiment, the chamber is a piston arrangement. Such devices are commercially available.

Alternatively, a plastic squeeze bottle with an aperture or opening dimensioned to aerosolize an aerosol formulation by forming a spray when squeezed is used. The opening is usually found in the top of the bottle, and the top is generally tapered to partially fit in the nasal passages for efficient administration of the aerosol formulation. Preferably, the nasal inhaler will provide a metered amount of the aerosol formulation, for administration of a measured dose of the drug.

The compositions may also be formulated in rectal or vaginal compositions such as suppositories or retention enemas, *e.g.,* containing conventional suppository bases such as cocoa butter or other glycerides.

In addition to the formulations described previously, the composition may also be formulated as a depot preparation. Such long acting formulations may be formulated with suitable polymeric or hydrophobic materials (for example as an emulsion in an acceptable oil) or ion exchange resins, or as sparingly soluble derivatives, for example, as a sparingly soluble salt.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer, Science 249:1527-1533, 1990, which is incorporated herein by reference.

The other therapeutics may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

The pharmaceutical compositions described herein contain an effective amount of the antibodies and optionally therapeutic agents included in a pharmaceutically-acceptable carrier. The term pharmaceutically-acceptable carrier means one or more compatible solid or liquid filler, diluents or encapsulating substances which are suitable for administration to a human or other vertebrate animal. The term carrier denotes an organic or inorganic ingredient, natural or synthetic, with which the active ingredient is combined to facilitate the application. The components of the pharmaceutical compositions also are capable of being commingled with the compounds described herein, and with each other, in a manner such that there is no interaction which would substantially impair the desired pharmaceutical efficiency.

The composition including specifically but not limited to the antibodies, may be provided in particles. Particles as used herein means nano or microparticles (or in some instances larger) which can consist in whole or in part of the antibody. The particles may contain the therapeutic agent(s) in a core surrounded by a coating, including, but not limited to, an enteric coating. The therapeutic agent(s) also may be dispersed throughout the particles. The therapeutic agent(s) also may be adsorbed into the particles. The particles may be of any order release kinetics, including zero order release, first order release, second order release, delayed release, sustained release, immediate release, and any combination thereof, etc. The particle may include, in addition to the therapeutic agent(s), any of those materials routinely used in the art of pharmacy and medicine, including, but not limited to, erodible, nonerodible, biodegradable, or nonbiodegradable material or combinations thereof. The particles may be microcapsules which contain the antibody in a solution or in a semi-solid state. The particles may be of virtually any shape.

Both non-biodegradable and biodegradable polymeric materials can be used in the manufacture of particles for delivering the therapeutic agent(s). Such polymers may be natural or synthetic polymers. The polymer is selected based on the period of time over which release is desired. Bioadhesive polymers of particular interest include bioerodible hydrogels described by H.S. Sawhney, C.P. Pathak and J.A. Hubell in Macromolecules, (1993) 26:581-587, the teachings of which are incorporated herein. These include polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

The composition may be contained in controlled release systems. The term "controlled release" is intended to refer to any drug-containing formulation in which the manner and profile of drug release from the formulation are controlled. This refers to immediate as well as non-immediate release formulations, with non-immediate release formulations including but not limited to sustained release and delayed release formulations. The term "sustained release" (also referred to as "extended release") is used in its conventional sense to refer to a drug formulation that provides for gradual release of a drug over an extended period of time, and that preferably, although not necessarily, results in substantially constant blood levels of a drug over an extended time period. The term "delayed release" is used in its conventional sense to refer to a drug formulation in which there is a time delay between administration of the formulation and the release of the drug there from. "Delayed release" may or may not involve gradual release of drug over an extended period of time, and thus may or may not be "sustained release."

Use of a long-term sustained release implant may be particularly suitable for treatment of chronic conditions. "Long-term" release, as used herein, means that the implant is constructed and arranged to deliver therapeutic levels of the active ingredient for at least 7 days, and preferably 30-60 days. Long-term sustained release implants are well-known to those of ordinary skill in the art and include some of the release systems described above.

Also provided herein are kits containing the antibody compositions. The kits include an antibody composition and may also contain one or more vials or containers. The kit may also include instructions for administering the component(s) to a subject who has a disease described herein, such as cancer, or who has symptoms of such a disease.

In some embodiments, the kit includes a pharmaceutical preparation vial, a pharmaceutical preparation diluent vial, and the antibodies. The vial containing the diluent for the pharmaceutical preparation is optional. The diluent vial contains a diluent such as physiological saline for diluting what could be a concentrated solution or lyophilized powder of the antibody. The instructions can include instructions for mixing a particular amount of the diluent with a particular amount of the concentrated pharmaceutical preparation, whereby a final formulation for injection or infusion is prepared. The instructions may include instructions for use in a syringe or other adminstration device. The instructions can include instructions for treating a patient with an effective amount of the antibodies. It also will be understood that the containers containing the preparations, whether the container is a bottle, a vial with a septum, an ampoule with a septum, an infusion bag, and the like, can contain indicia such as conventional markings which change color when the preparation has been autoclaved or otherwise sterilized.

The present invention is further illustrated by the following Examples, which in no way should be construed as further limiting.

Examples

1. Generation of transgenic goats expressing highly galactosylated antibodies

*Generation of CD20 antibody constructs*

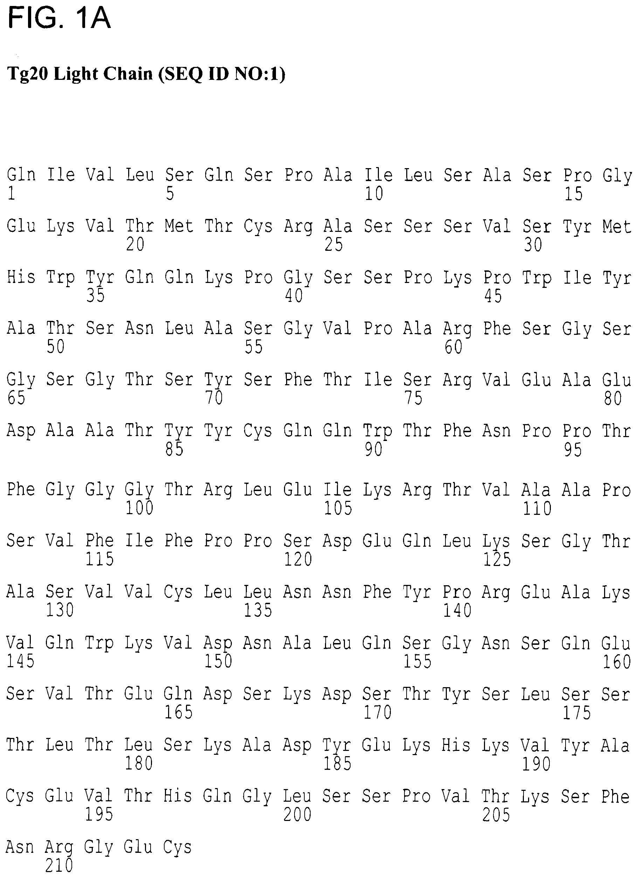

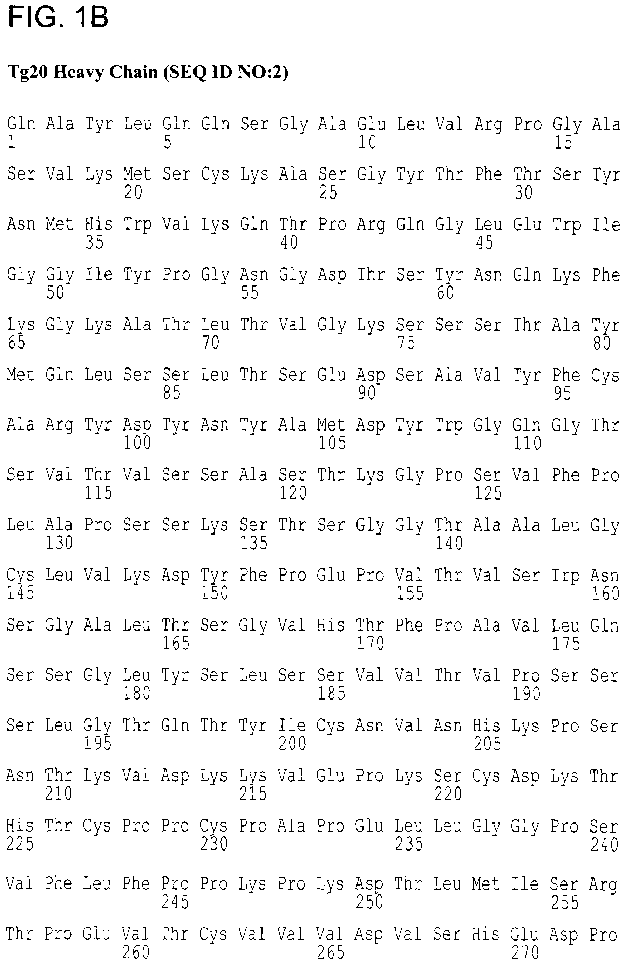

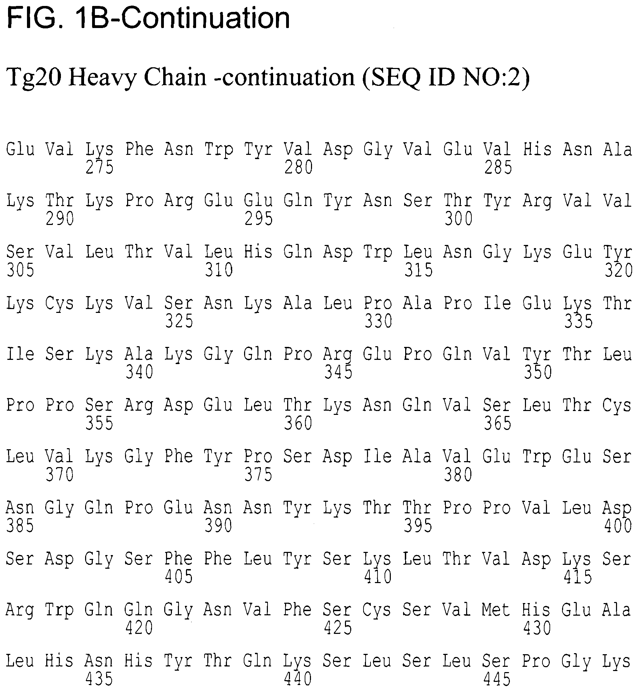

The amino acid sequence for the transgenic CD20 antibody (Tg20) is provided in **Figure 1:** the light chain is SEQ ID NO:1 and heavy chain is SEQ ID NO:2. The nucleic acid sequence encoding the amino acid sequence for the transgenic CD20 antibodies was used to generate transgenic goats expressing the transgenic CD20 antibody (Tg20). A casein promoter was operably linked to the nucleic acid sequence encoding the amino acid sequence for the transgenic CD20 antibodies to facilitate expression of the nucleic acid sequence in the mammary gland of the goat

The nucleic acid sequences coding for the light and heavy chains of the anti-CD20 antibody were synthesized with the following additions/changes:

1) flanking XhoI sites were added to facilitate subcloning into the expression vector Bc800.
2) A Kozak consensus sequence (GCCACC) was added immediately upstream of the initiator ATG on both constructs.
3) A silent mutation was introduced near the termination codon of the heavy chain to destroy a potential splice site: **G GGT** AAA TGA (SEQ ID NO:3) to **G GGA** AAA TGA (SEQ ID NO:4).

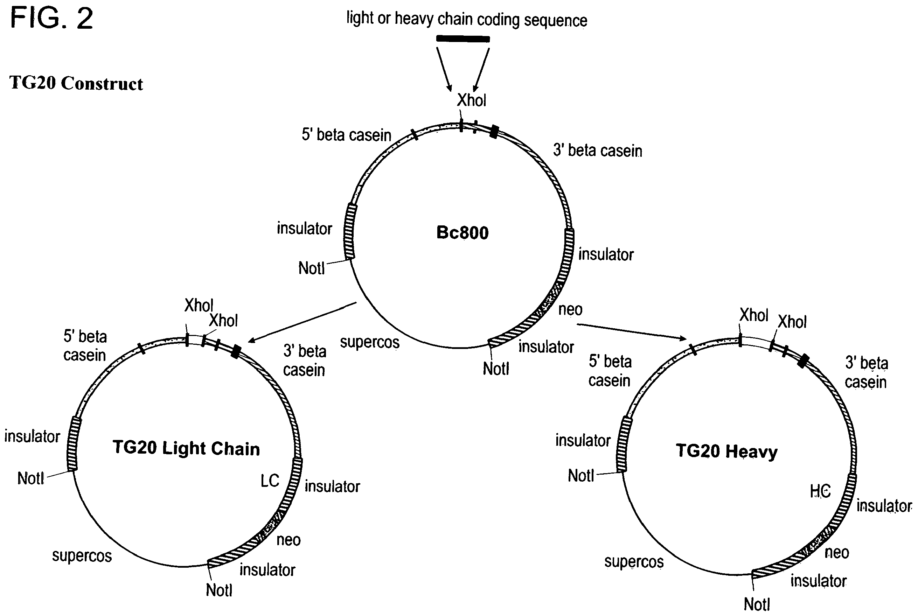

Both the light and heavy chain sequences were subcloned into the XhoI cloning site of the expression vector Bc800, which contains a chicken beta globin insulator sequence, 6.2 kb of 5' beta casein promoter sequence, an XhoI cloning site, 7.1 kb of 3' beta casein downstream sequence, another insulator sequence, G418 resistance marker, and a final insulator sequence, in a SuperCos backbone. (See **Figure 2**). Plasmid DNA was prepared by cesium chloride centrifugation. The SuperCos backbone was released using flanking NotI sites and separated from the transgene fragment by electrophoresis through agarose gel. The resulting purified nucleic acid fragment was then used for somatic cell nuclear transfer. The cDNA fragments encoding the heavy and light chains of TG20 were inserted into a mammary specific expression vector to obtain 2 transgenes which co-transfected into female goat fetal cells (LipofectAMINE, Gibco). Nuclear transfers were performed as described previously (Melican et al. 2005; Theriogenology 63:1549).

*Production of Skin Fibroblast Lines*

Fibroblasts from fresh goat skin biopsy samples were maintained in primary culture *in vitro*. Briefly, skin samples were minced in $Ca^{++}$-free and $Mg^{++}$-free phosphate buffered saline (PBS), harvested with dilute trypsin in EDTA to recover single cell suspensions and cultured at 37°C. Confluent cells were trypsinized and sub-cultured. Aliquots of cells were cryopreserved in liquid nitrogen for future use.

*Analysis of Transfected Cell Lines*

Transfected cells were characterized by Southern blot analysis with probes specific for the transgenes, to establish the transgene copy number and identify potential rearrangements. Each cell line also was analyzed by FISH to confirm single integration and to determine chromosomal location. Cytogenetic analysis was performed to confirm the karyotypes of the cell lines. PCR, southern blot and FISH analysis confirmed the presence of both heavy and light Ig chain transgenes.

*FISH*

For Interphase FISH, a few hundred cells from each expanded colony were immobilized on filters and hybridized to amplified transgene-specific digoxigenin-labeled probes. For metaphase FISH, cells were cultured on Lab Tek Chamber slides (Nunc, Rochester, NY) and pulsed with 5-bromo-2'deoxyuridine (BrdU) to allow for replication banding. Probe binding was detected with FITC-conjugated anti-digoxigenin, and the chromosomes were counterstained with 4',6-Diamidino-2-phenylindole (DAPI). Images were captured using a Zeiss Axioskop microscope (Zeiss Imaging, Thornwood, NY), a Hamamatsu digital camera (Hamamatsu, Bridgewater, NJ), and Image Pro-Plus software (Media Cybernetics, Silver Springs, MD).

*Cytogenetic Analysis*

Cytogenetic analysis was performed on donor transfected fibroblast cell lines. Transgene probes were labeled with digoxigenin-dUTP by nick translation. Probe binding to the denatured chromosomes were detected either with FITC-conjugated anti-digoxigenin or with horseradish peroxidase-conjugated anti-digoxigenin followed by FITC-conjugated tyramide. Chromosome banding patterns were visualized with DAPI. Goats have 60 chromosomes, all of them acrocentric (having the centromere at one end rather than at or near the middle). The metaphase spreads were inspected for evidence of gross abnormalities such as chromosome loss, duplication or gross rearrangement.

*Generation of Transgenic Animals Expressing CD20 antibodies*

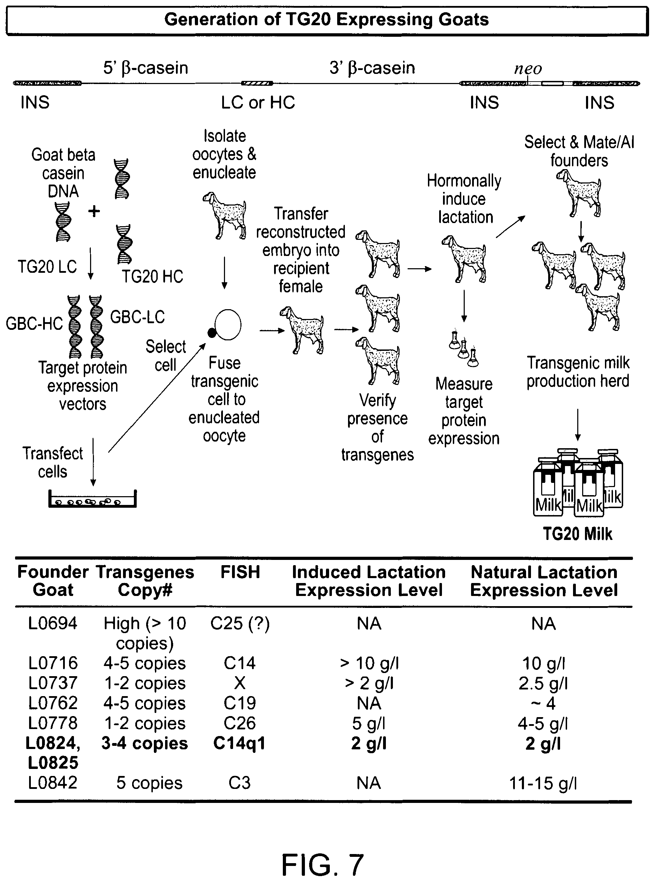

Transgene constructs for the CD20 antibodies were used to generate transgenic goats. Transgenic goats producing mature antibodies were generated by introducing a 1:1 mixture of heavy chain and light chain constructs.
Transgenic goats with pre-defined genetics were generated using nuclear transfer techniques routine in the art. The transgenic construct described above was introduced into skin fibroblast cell lines by standard transfection. The recombinant primary cell lines were screened *in vitro* for transgene copy-number, integrity and integration site, before they were used to produce transgenic animals.
Goats were maintained at a USDA registered, FDA and EMA inspected facility. Transgene analysis of offspring (FISH, PCR and Southern blots) was conducted using genomic DNA isolated from blood and tissue samples. Transgene analysis of offspring (FISH, PCR and Southern blots) was conducted using genomic DNA isolated from blood and tissue samples (See **Figure 7).**

*Purification of Antibodies*

Antibodies were harvested from the milk of transgenic goats. Goat milk was from hormonally induced lactations of transgenic goats (See *e.g.,* Ebert et al., 1994, Biotech 12: 699-702). The milk was clarified to remove the majority of the casein and fat by centrifugation. The antibodies were then purified by Protein A chromatography followed by anion exchange chromatography on Q Sepharose Fast Flow. The final product was formulated in sodium citrate/sodium chloride + polysorbate 80.

2. Analysis of trans genically produced antibodies

*A. Materials and Methods:*

*Glycosylation profiling*

*N*-deglycosylation of the antibody samples was carried out according to the manufacturer's procedure using a Prozyme *N*-deglycosylation kit (San Leandro, CA, USA). Briefly, 300 $\mu$g of dried antibody sample were recovered in

135 μL of a 10-mM aqueous Tris-HCl buffer pH 8.0, and 4.5 μL of a 10% (v/v) β-mercaptoethanol aqueous solution was added to reduce the antibody disulfide bridges. The *N*-deglycosylation was carried out by the addition of 7.5 mU of peptidyl-N-glycosidase (PNGase) F followed by an overnight incubation at 37°C.

At this stage, many *N*-glycans were released as glycosylamines before slowly hydrolyzing into reducing glycans. The full regeneration of reducing glycans was performed by adding to PNGase F-digested antibody samples glacial acetic acid at a final concentration of 5% (v/v) followed by a one hour incubation at room temperature. The freshly regenerated reducing N-glycan mix was purified by a solid phase extraction (SPE) onto a 50-mg Hypersep Hypercarb porous graphitized carbon (PGC) column (Thermofischer Scientific, Bremen, Germany) (Packer et al., 1998). The PGC SPE column was sequentially washed with 1 mL methanol and 2 x 1 mL of a 0.1% (v/v) aqueous trifluoroacetic acid (TFA). The oligosaccharides were dissolved in 200 μL of a 0.1% (v/v) aqueous TFA, applied to the column and washed with 2 x 1 mL of a 0.1% (v/v) aqueous TFA. The elution of the glycans was performed by applying 2 x 400 μL of a 25% (v/v) aqueous acetonitrile containing 0.1% (v/v) TFA and the eluate was vacuum-dried.

The PGC-purified glycans were reductively aminated with 2-aminobenzamide (2-AB) by recovering dried glycans by 10 μL of a 33% (v/v) acetic acid in DMSO containing 0.35 M 2-AB and 1 M sodium cyanoborohydride and the reaction was kept at 37°C for 16 hours. The 2-AB-labeled N-glycans were purified onto a 50-mg Oasis polymeric HLB SPE column, used in the hydrophilic interaction chromatography (HILIC) mode (Waters, Milford, MA, USA). The HILIC SPE column was sequentially wetted with 1 mL of a 20% (v/v) aqueous acetonitrile and equilibrated with 2 x 1 mL of acetonitrile, the 2-AB derivatives dissolved in acetonitrile were then loaded onto the SPE column. After washing the column with 2 x 1 mL of acetonitrile, the elution of the 2-AB derivatives was next performed by applying 2 x 500 μL of a 20% (v/v) aqueous acetonitrile. The 1-mL eluate was vacuum-concentrated to 50 μL.

The purified 2-AB derivatives were finally profiled by normal-phase high-performance liquid chromatography (NP-HPLC) using a 150 x 4.6 mm ID TSK-gel amide-80 HILIC HPLC column (TOSOH Bioscience, King of Prussia, PA, USA) with 3 μm packing particules (Guile et al., 1996). The mobile phase was composed of a mixture of a 50-mM ammonium formate aqueous solution adjusted at pH 4.4 (A) and acetonitrile (B). The operating flow rate and temperature were respectively 1 mL/min and 30°C. 5 μL of the purified 2-AB derivatives were 40-fold diluted using a 80% (v/v) aqueous acetonitrile, and 50 μL the freshly shaken organic mixture were injected to the HILIC column, equilibrated with 80% (v/v) B. Once sample injected, the separation of the N-glycans were performed as following: from 80% to 70% (v/v) B in 15 min; from 70% to 55% (v/v) B in 150 min; from 55% to 10% (v/v) B in 5 min; 10% (v/v) B during 10 min; from 10% to 80% (v/v) in 1 min; 80% (v/v) B during 45 minutes (reequilibration). The detection of the fluorescent derivatives was performed by fluorescence detection (FD) with an excitation wavelength of 330 nm and an emission wavelength of 420 nm.

References: Guile GR, Rudd PM, Wing DR, Prime SB, Dwek RA. A rapid high-resolution high-performance liquid chromatographic method for separating glycan mixtures and analyzing oligosaccharide profiles. Anal Biochem. 1996 Sep 5; 240(2):210-26; Packer NH, Lawson MA, Jardine DR, Redmond JW. A general approach to desalting oligosaccharides released from glycoproteins. Glycoconj J. 1998 Aug; 15(8):737-47.

*Binding to human CD16a*

Binding to human CD16a was performed using Surface Plasmon Resonance (SPR) technology on a Biacore system (X100, GE Healthcare). In this assay, the CD16a was immobilized on a SPR chip using the amine chemistry at a level of 2183 RU. The antibody to be tested was diluted in PBS buffer at different concentrations (50, 100 and 200 nM) and injected sequentially on the immobilized CD16a with the same buffer. On the chip, one flow-cell was used as a control in order to subtract background caused by non-specific interactions. Between each injection a regeneration of the chip was performed with a 3.75 nM NaOH solution.

*CD16 assay*

Briefly, NK effector cells have been substituted by immortalized transgenic cellular cells (Jurkat cells expressing human CD16a) to allow high reproducibility of the experiments. Jurkat CD16a cells, WIL2-S cells and PMA (Phorbol-Myristate Acetate) were used respectively as effector cells, target cells and non-specific activator and were incubated with a dose range of the antibody to be tested. After incubation, the Jurkat cell activation resulted in IL-2 cytokine release, quantified by a specific ELISA (Vivier E et al., Int. Immunol. 1992, 4 (11):1313-1323). The amount of IL-2 in the supernatant cell culture is directly correlated to the ability of WIL2-S/antibody to be tested immune complexes to bind and activate CD16a.

*ADCC assay*

ADCC (Antibody-Dependent Cellular Cytotoxicity) used to test the pharmacological activity of the antibody to be tested was realized by the lactate dehydrogenase release assay. Human NK cells from healthy donors, used as effector cells, were purified using immunomagnetic cells sorting with NK cell isolation kit and AutoMacs automat (Miltenyi biotec). Burkitt's lymphoma Raji cell lines (ATCC CCL 86), maintained in culture in IMDM containing 10% FCS (PAA The cell culture company, Les mureaux, France) were used as target cells. Cells were co-incubated at an effector/target ratio R=10/1 in the presence of different concentrations of antibody in microplates, and incubated in humidified atmosphere containing 5% $CO_2$ at 37°C for 4 hours. Negative control wells, comprising a well containing only the target cells and another one with only the effector cells, were included in the test to enable distinguishing from spontaneous effector cell death *versus* effector cell-mediated target cell death. An "antibody control" well, prepared by co-incubating the target cells and the antibody, was also run to test the intrinsic cytotoxic property of the antibody toward target cells in the absence of effectory cells. Finally, a control well was prepared by co-incubating both the target and effectory cells in the absence of the antibody in order to evaluate a potential antibody-independent cellular cytotoxicity (AICC) activity. After centrifugation, supernatants were tested for LDH release using Cytotoxicity detection kit (Roche Applied Science, Germany).

After thawing, PBMC were washed and suspended in RPMI supplemented with 10% FCS. MEC-1 or SUDHL-8 cell lines were incubated with human mAb at 20 microg/ml for 30 minutes at 4 °C. After washing, cell lines were labeled with carboxyfluorescein diacetate succinimidyl ester (CFSE) for 10 minutes. Labeled target cells were suspended in RPMI 1640 (+10% FCS) and mixed with PBMC at various effector/target (E/T) ratios. Cells were incubated 4 hours at 37 °C, and analyzed by FC after staining with PI. Human B-CLL or human B lymphocytes were enriched from PBMC from CLL patients or healthy volunteers using kits from Miltenyi.

The ADCC activity (%) for each antibody concentration was calculated according to the formula: [(antibody + Target + NK) - (antibody + target)] / [100 - (antibody + target)] - [(NK + target) - (antibody + target)] / [100- (antibody + target)]. The experimental values were exploited using Prism software (Graphpad Software Inc., La Jolla, CA, USA) and fit to a sigmoidal dose-response curve, and the $E_{max}$ (maximum cytotoxicity) and $EC_{50}$ (antibody concentration required in order to obtain 50% of the $E_{max}$) were determined.

*CDC assay*

The antibody to be tested was mixed at one concentration with WIL2-S cells expressing the CD20 antigen in presence of human serum. In each test, eight samples were prepared independently. Cells in human C1q depleted serum were spiked 10 minutes at 37 °C with human mAb at 10 microg/mL and C1q at various concentrations. Cell death was determined by intercalation of the DNA dye propidium iodide (PI) by fluorescence. The fluorescence level is proportional to the number of viable cells in the culture medium (O'Brien, J. et al (2000) Eur. J. Biochem. 267, 5421-5426)

*Cynomolgus pharmacokinetic study*

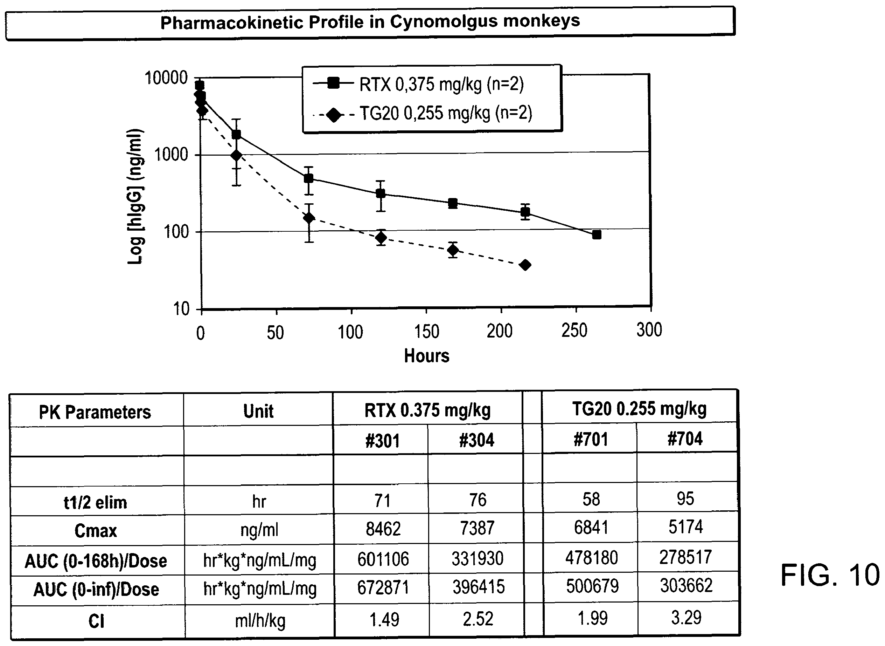

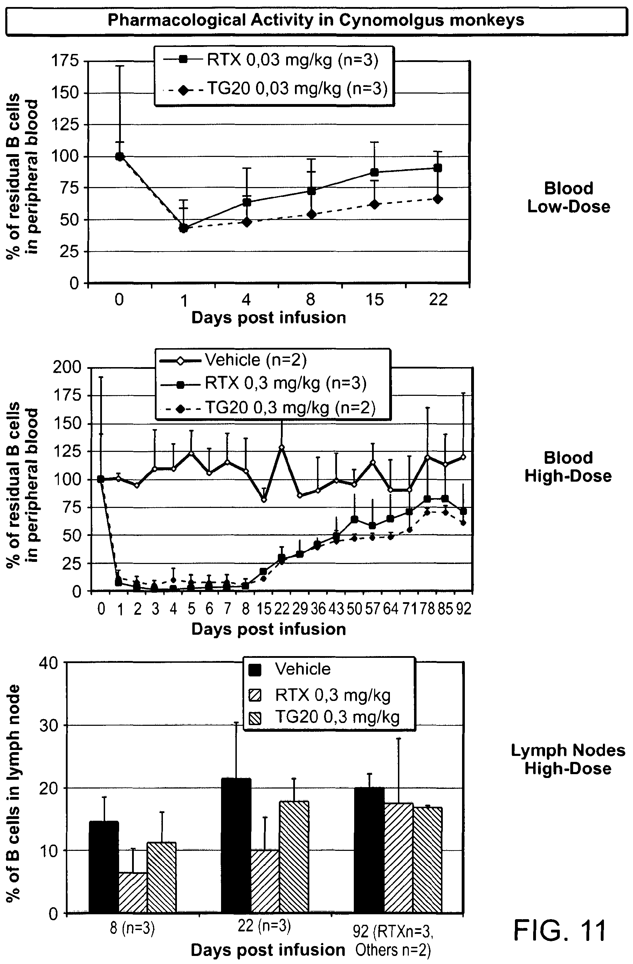

Cynomolgus monkeys (*Macaca fascicularis*) ranged in weight from 3.1 to 4.4 kg. Before dosing initiation, all animals were weighed and assigned to treatment groups using a computerized randomization procedure. Dosing formulations were administered IV. Blood samples were collected twice before initiation of dosing (including spare animals) with the second sampling collected between 1 and 4 days before dosing, then on Day 1 (4 hours postdose), and on Days 2 to 8, 15, 22, 29, 36, 43, 50, 57, 64, 71, 78, 85, and 92. Animals were euthanized on Day 92. The blood samples were analyzed using a qualified analytical method. The lymphocytes populations were quantified (by flow cytometry, using specific antibodies against cell surface markers. Lymph nodes (LN) were collected by excisional biopsy, the lymphocytes populations were quantified as relative percentage of CD45+ lymphocytes by flow cytometry, using specific antibodies against cell surface markers. Pharmacokinetic parameters were estimated using WinNonlin pharmacokinetic, software (Pharsight Corp., Mountain View, California). A non-compartmental approach was used for parameter estimation (See **Figure 10** and **Figure 11**).

*Whole Blood Experiments*

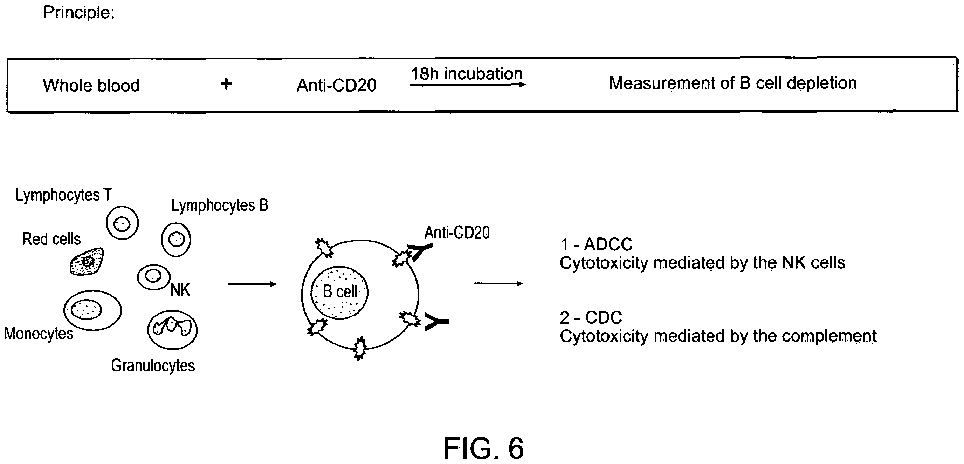

This test was performed to assess the ability of Tg20 to induce depletion of B lymphocytes in whole blood of human donors by all natural immunologic phenomena in particularly CDC and ADCC according to **Figure 6.**

The activity of antibodies in B cell depletion (in peripheral blood and in some lymphoid organs) and the pharmacokinetics profile was monitored for different anti-CD20 antibodies: RTX, and TG20 in cynomolgus monkeys when administered by the intravenous route at two different doses.

The study design was as follows:

- Dose levels: 0.03 mg/kg/day (low) and 0.3 mg/kg/day (high)
- Three animals per dose per antibody

*Blood phenotyping*

Blood samples were collected twice before initiation of dosing (including spare animals) with the second sampling collected between 1 and 4 days before dosing, then on Day 1 (4 hours postdose), and on Days 2 to 8, 15, 22, 29, 36, 43, 50, 57, 64, 71, 78, 85, and 92. Blood samples were collected at approximately the same time during Days 2 through 92. A volume of 1 mL of blood was collected from the femoral vein into tubes containing $K_2$EDTA as anticoagulant for analysis of lymphocyte subsets. Samples were not collected from the animals at scheduled termination following Day 22. Samples were mixed gently and kept at ambient conditions until transferred to the Immunology laboratory at the Testing Facility for processing.
The blood samples were analyzed using a qualified analytical method. The lymphocytes populations identified in the following table were quantified (relative percentages and absolute
counts) by flow cytometry, using specific antibodies against cell surface markers. Samples were
processed using the following antibody panels: CD45/CD3/CD8/CD4, CD45/CD3/CD8/CD16,
and CD45/CD3/CD20/CD40 up to Day 22. Then from Day 29 to Day 92 only the B lymphocytes antibody panel was
assessed (CD45/CD3/CD20/CD40). The total lymphocyte
counts was determined for each antibody panel tube, using BD TruCount tubes, and reported as
the mean of CD45+ lymphocytes per microL of whole blood for each sample. Isotype controls were not used and the DPBS tube was used as the negative control.

*Lymph Node Immunophenotyping:*

Right inguinal lymph nodes (LN) were collected by excisional biopsy from animals on Day 8, and left inguinal lymph nodes were collected by excisional biopsy on Day 22. The right axillary lymph node was collected by clean removal from on Day 92 (at necropsy). The animals were food deprived overnight prior to scheduled surgery. Carprofen (4 mg/kg) was given subcutaneously to each animal at least 30 minutes prior to the surgery. Each animal received an intramuscular injection of Duplocillin® (1 mL) before the surgery and 2 days after. Each animal was preanesthetized with an intramuscular injection of ketamine, xylazine and glycopyrrolate to achieve sufficient sedation prior to presurgical preparation. The animal underwent tracheal intubation and thereafter anesthesia was maintained using isoflurane and oxygen as per SOP Pre-anesthesia and Anesthesia. A pulse oximeter was used to monitor heart rate and O2 saturation. Prior to surgery, a bland lubricating ophthalmic agent was administered to each eye. Once the animal was sufficiently anesthetized, the right or left inguinal area was shaved and prepared as per SOP on surgical site preparation. An incision of approximately 2-3 cm was made in the right or left inguinal area. The surrounding tissues were carefully dissected in order to visualise the right or left inguinal lymph nodes. Using scissors and forceps, the lymph nodes were collected. A saline flush of 10 mL of warm saline was given prior to wound closure. After collection, the subcutaneous tissue and skin were closed using absorbable suture. The lymph nodes were kept at ambient room temperature into approximately 5 mL of assay medium (RPMI-1640 containing 5% (v/v) FBS) and transferred to the Immunology laboratory until processing/analysis. Populations identified in the above text table were quantified as relative percentage of CD45+ lymphocytes by flow cytometry, using specific antibodies against cell surface markers. Samples were processed using the following antibody panels: CD45/CD3/CD8/CD4, CD45/CD3/CD8/CD16 and CD45/CD3/CD20/CD40. Isotype controls were not used and the DPBS tube was used as the negative control.

*B: Results*

*SDS PAGE:*

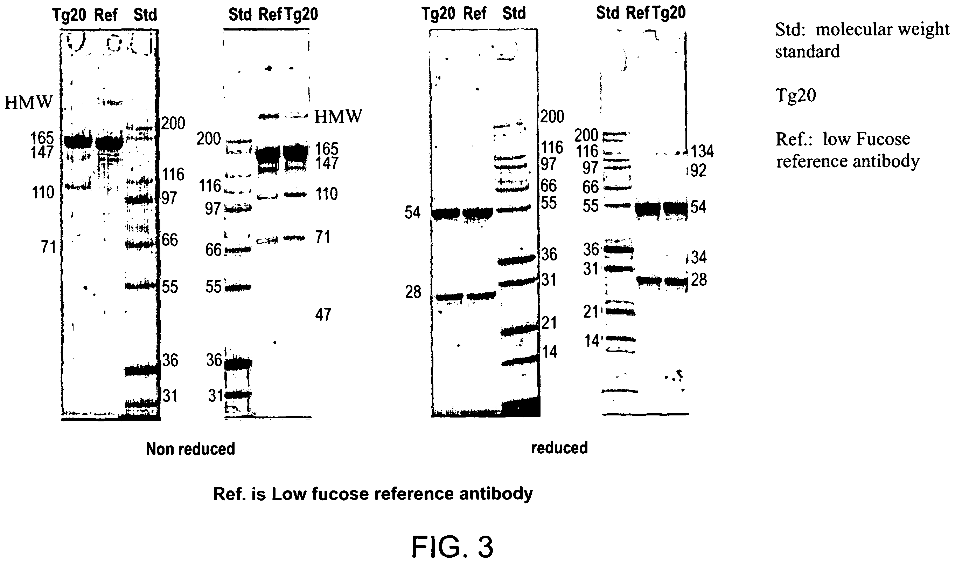

SDS-PAGE analysis performed under non-reducing conditions and after Coomassie blue staining (see **Figure 3**), shows a major band at 168 kDa for both Tg20 and the low fucose reference antibody, corresponding to the intact antibody. A band of high molecular weight (HMW) was detected for the reference antibody whereas it was not observed for Tg20. After silver staining, this HMW band was also detected for Tg20 as well as several other minor bands commonly detected for the reference antibody. These minor bands could correspond to partially reduced forms of the antibody: 147 kDa for HC-HC-LC, 110 kDa for HC-HC and 71 kDa for HC-LC. The major band at 168 kDa was estimated at 95% of the total protein content while the other bands of product-related impurities represent only 5%, suggesting a low level of degradation for Tg20. SDS-PAGE analysis performed under reducing conditions (see **Figure 3**) shows two major bands at 54 and 28 kDa corresponding respectively to the heavy and light chains of the antibodies. The two antibodies exhibit the same apparent molecular masses.

*Glycosylation profiling*

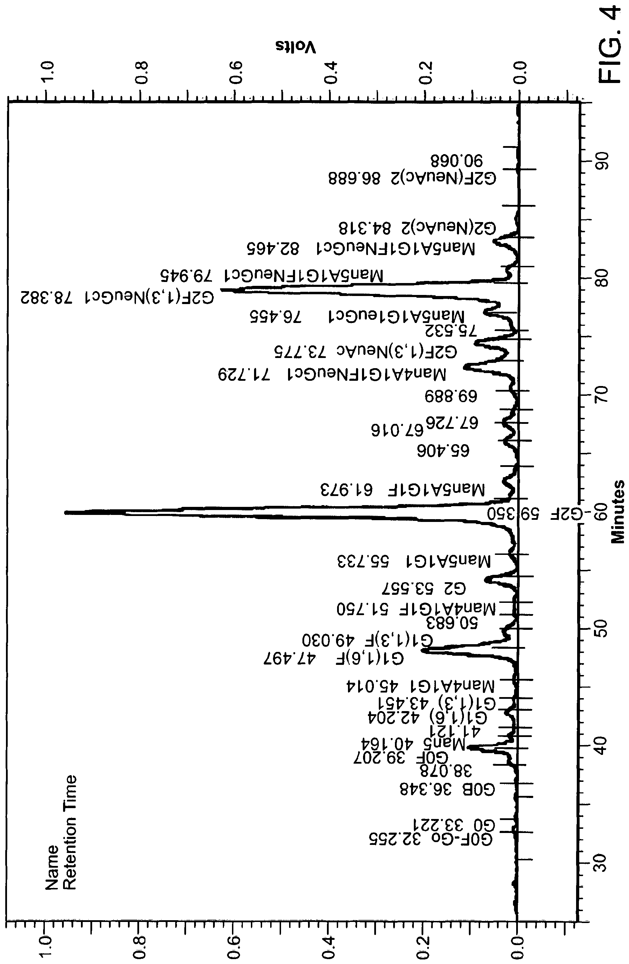

**Figure 4** shows the NP-HPLC profile obtained by fluorimetric detection of 2-AB derived N-glycans released from Tg20 by a PNGase F treatment. The major peaks detected at 47.49, 59.35 and 78.38 min correspond respectively to A2G1F, A2G2F and A2G2FNeuGc1. Numerous lower abundant peaks were also detected. The relative molar ratio of the detected forms are displayed in **Table 1** (See below).

In this experiment, the level of sialylated structures was estimated at 43%. The level of high mannose-hybrid structures was estimated at 15% versus 85% for complex structures. The overall galactosylation level was of 91% and the fucosylation level was estimated at 92%.

**Table 1**: relative molar ratios of major N-glycans released from TG20 and analyzed by NP-HPLC-FD.

| NAME | RT | AREA | REL AREA |
|---|---|---|---|
| G0F-Gn | 32,3 | 372525 | 0,3 |
| G0 | 33,2 | 126744 | 0,1 |
| G0B | 36,3 | 41050 | 0,0 |
| G0F | 39,2 | 4045579 | 3,0 |
| Man5 | 40,2 | 550140 | 0,4 |
| A2G1 | 42,2 | 1046877 | 0,8 |
| A2G1 | 43,5 | 210278 | 0,2 |
| Man4A1G1 | 45,0 | 375461 | 0,3 |
| A2G1F | 47,5 | 10693605 | 7,9 |
| A2G1F | 49,0 | 1621545 | 1,2 |
| Man4A1G1F | 51,8 | 314927 | 0,2 |
| A2G2 | 53,6 | 3235548 | 2,4 |
| Man5A1G1 | 55,7 | 1026319 | 0,8 |
| A2G2F | 59,4 | 51192671 | 37,9 |
| Man5A1G1F | 62,0 | 1949522 | 1,4 |
| Man4A1G1FNeuGc1 | 71,7 | 7515884 | 5,6 |
| A2G2FNeuAc1 | 73,8 | 5174288 | 3,8 |
| Man5A1G1NeuGc1 | 76,5 | 4047105 | 3,0 |
| A2G2FNeuGc1 | 78,4 | 36337467 | 26,9 |
| Man5A1G1FNeuGc1 | 80,0 | 1411462 | 1,0 |
| Man5A1G1FNeuGc1 | 82,5 | 3105914 | 2,3 |
| A2G2NeuAc2 | 84,3 | 456704 | 0,3 |
| A2G2FNeuAc2 | 88,7 | 325766 | 0,2 |
| TOTALS | | 135177381 | 100,0 |

| | | |
|---|---|---|
| Highmannose/Hyb level (%) | | 15 |
| Fucosylation level (%) | | 92 |
| Bisecting GlcNAc level (%) | | 0 |
| Level of galactosylated structures | | 96 |
| Galactosylation level (%) | | 91 |
| level of Sialylated structure (%) | | 43 |

*Primary structure*

The protein sequence has been investigated by protein and peptide mapping. A sequence coverage of 93% was obtained by peptide mapping and the molecular weight of heavy and light chains deduced from protein mapping experiment complies with theoretical masses. Both heavy and light chains have been identified as pyroglutaminated at their N-terminal glutamine. The N- and C-terminal ends of both the heavy and light chains were confirmed.

*Size exclusion chromatography*

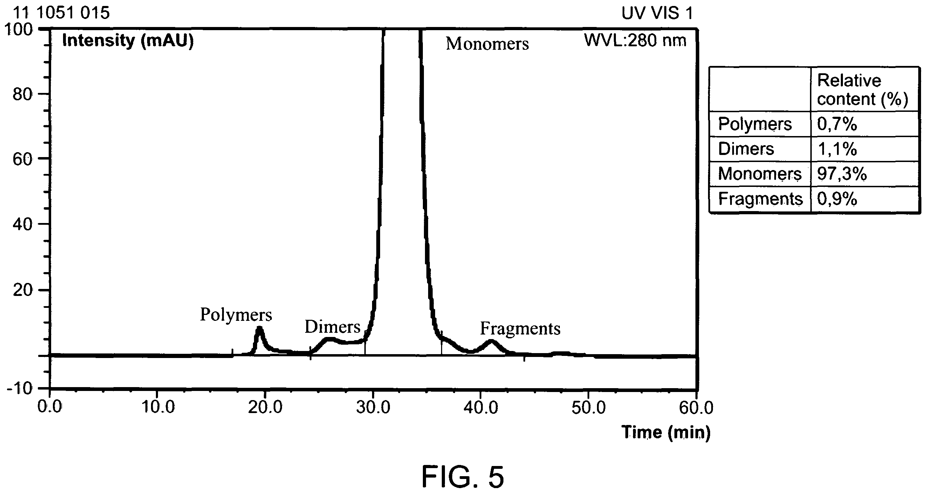

Study of Tg20 size exclusion chromatography (SEC) profile shows that the monomeric form of the product represents 97.3 % of the detected forms **(Figure 5).** Fragments, dimers and polymers were detected in low abundance, at 0.9 %, 1.1 % and 0.7 %, respectively.

*Binding to human CD16a*

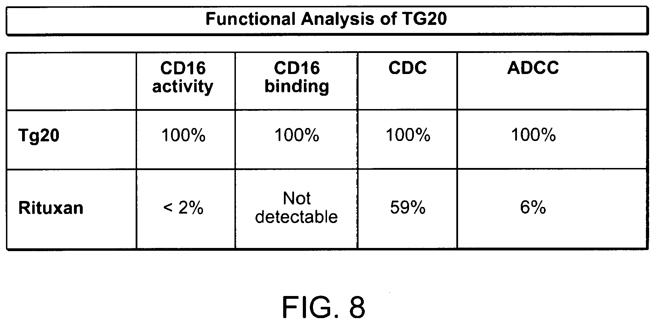

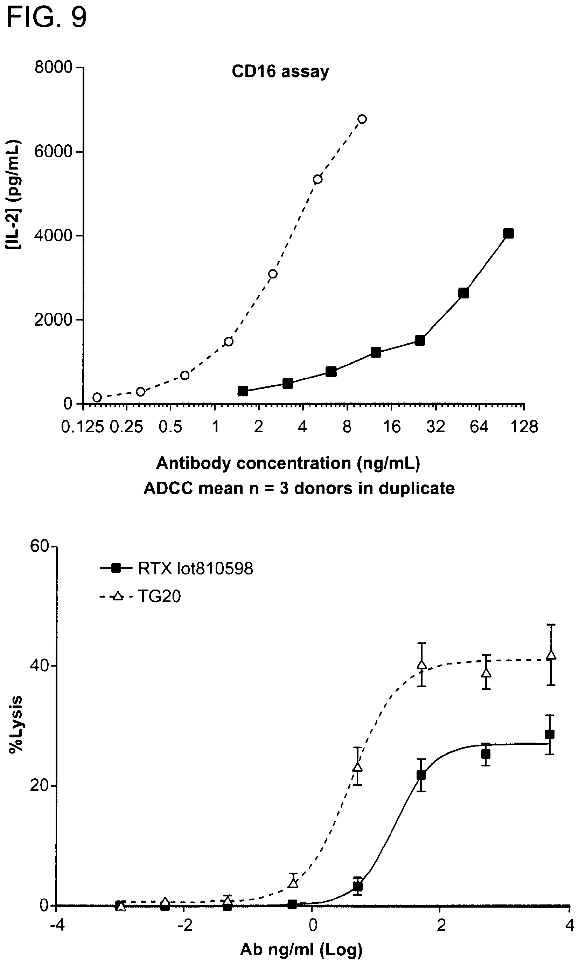

This assay has been developed to focus on the ability of an antibody to bind the CD16a which is associated with its ADCC activity. The interaction curves obtained for Tg20 show that TG20 has an ability to bind the immobilized CD16a. Rituxan displayed a non-detectable profile by SPR in the same experimental conditions (See **Figure 8** and **Figure 9**).

*CD16 activation by monoclonal antibody CD20 assay*

This assay was developed to assess the ability of Tg20 bound on CD20 target cells (WIL2-S cells) to activate effector cells expressing on their surface the FcγRIIIa receptor (also called CD16a). This binding triggers the effector cells activation and IL-2 release. This assay evaluates the potency of anti-CD20 antibodies to induce ADCC mediated by Natural Killer (NK) cells.

The results, expressed as the amount of IL-2 cytokine released, showed a significant activity of Tg20. The activity of Tg20 was significantly higher than Rituxan (See **Figure 8** and **Figure 9**).

*ADCC experiments*

This test assesses the ability of Tg20 bound on CD20+ target cells (Raji cells) to activate NK cells and to induce target cell lysis. The cytotoxic activity mediated by the anti-CD20 antibodies (Tg20 and Rituxan) is expressed as % of lysis of target cells.

The results of these assays show that the cytotoxic activity induced by Tg20 was significantly higher than the cytotoxic activity induced by Rituxan (See **Figure 8** and **Figure 9**).

*CDC assay*

This assay is based on the ability of the antibody to activate the complement system when the IgG is linked to its target. Tg20 showed higher CDC activity than Rituxan (See **Figure 8**).

*Whole Blood Experiments*

In the Whole blood experiment, Tg20 induced higher B cell depletion levels as compared to Rituxan.

The following parameters and endpoints were evaluated in this study: clinical signs, body weights, body weight changes, appetence, immunophenotyping (blood and lymph node), immunogenicity, toxicokinetic parameters, and gross necropsy findings.

Administration of RTX, or TG20 did not result in any unscheduled deaths, clinical observations, effects on body weight and appendence, or macroscopic changes at either dose level during the study.

Based on the immunophenotyping results, it was determined that a single injection of RTX, and TG20 resulted in a dose-dependent depletion of the B lymphocytes in blood. At the low dose and when compared to the pre-dosing levels, the depletion of B lymphocytes was slightly more pronounced in the group dosed with RTX (70%) as compared to the group dosed with TG20 (60%). At the high dose, the depletion of B lymphocytes was comparable. The B lymphocyte population recovered earlier with RTX (Day 3; low dose, Day 50; high dose) than with TG20 (Day 22; low dose, Day 78; high dose).

The incidence of full depletion of the B lymphocytes (less than 100 cells/microL of blood) at the high dose level was greater with RTX (3/3 animals) than with than with TG20 (1/3 animals). The incidence of B lymphocyte depletion and its recovery in blood correlated with the depletion of B lymphocytes observed in the lymph nodes on Day 8 and the kinetics of recovery on Days 22 and 92.

In the blood, a test article-related decrease of the NK lymphocytes was noted with RTX and TG20 at the low and high doses, with a recovery by Day 22. A test article-related decrease of T lymphocyte counts was noted on Day 1 or 2 with the high dose of RTX. The decrease was slight in magnitude and was recovered on Day 3 for most of the animals. No clear trend towards a decrease was observed for the T lymphocytes absolute counts of animals dosed with TG20. As a result of the decreases observed for the aforementioned cell subsets in the blood, a dose dependent decrease of the total lymphocyte counts was noted with the 3 compounds. At the low dose, the decrease in total lymphocyte counts vs. baseline levels was more pronounced in animals dosed with TG20 (Day 1: 57%) as compared to RTX (Day 1: 51%). At the high dose, the decrease in total lymphocyte counts was more apparent with RTX (Day 1: 70%) than with TG-20 (Day 1: 60%). The recovery was similar with the 3 compounds (partially to fully recovered by Day 22). In lymph nodes, no test article-related change was observed in the T and NK lymphocyte subsets on Days 8 and 22.

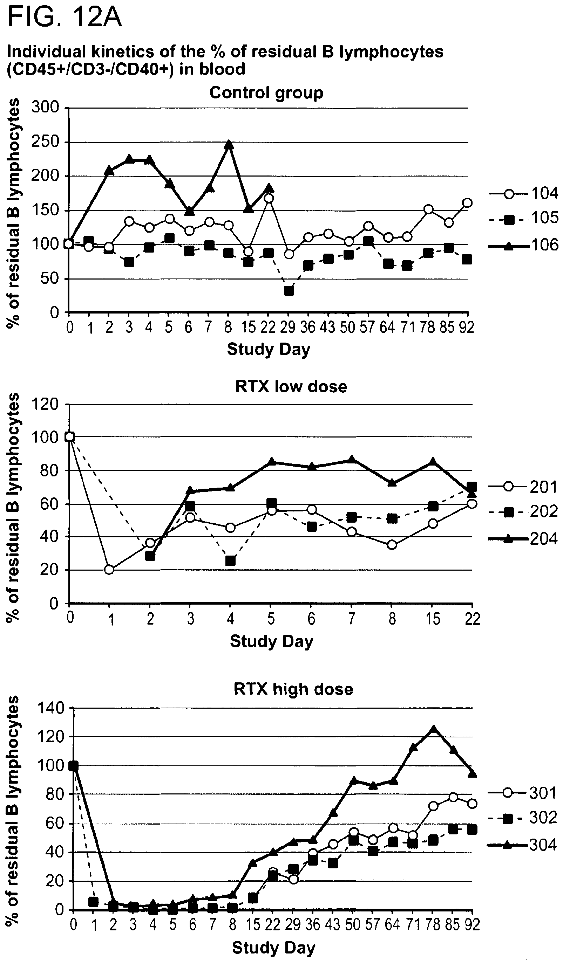

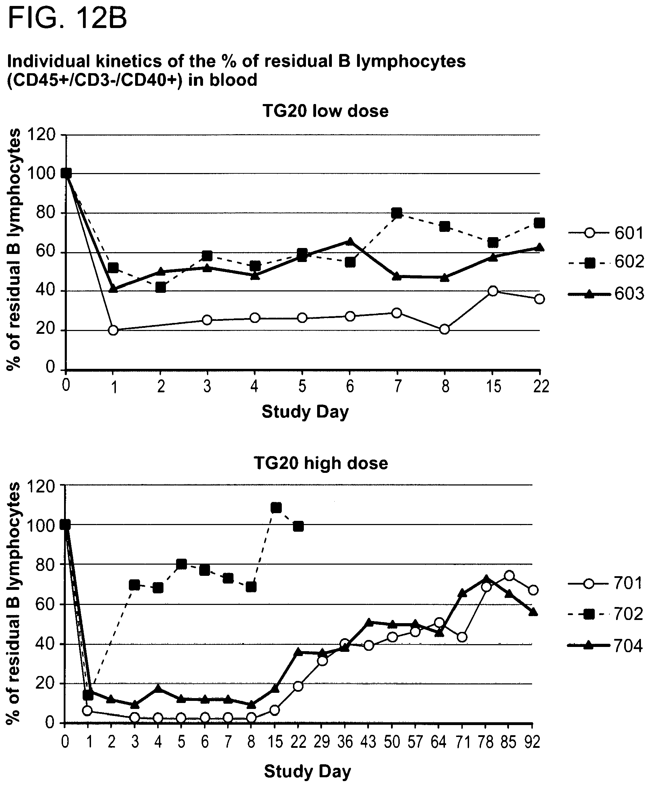

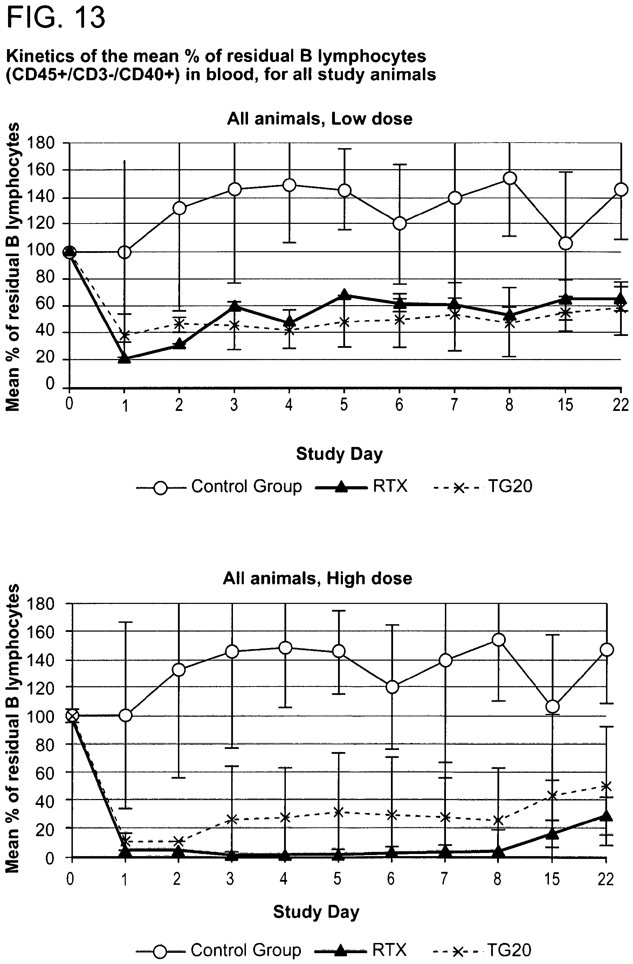

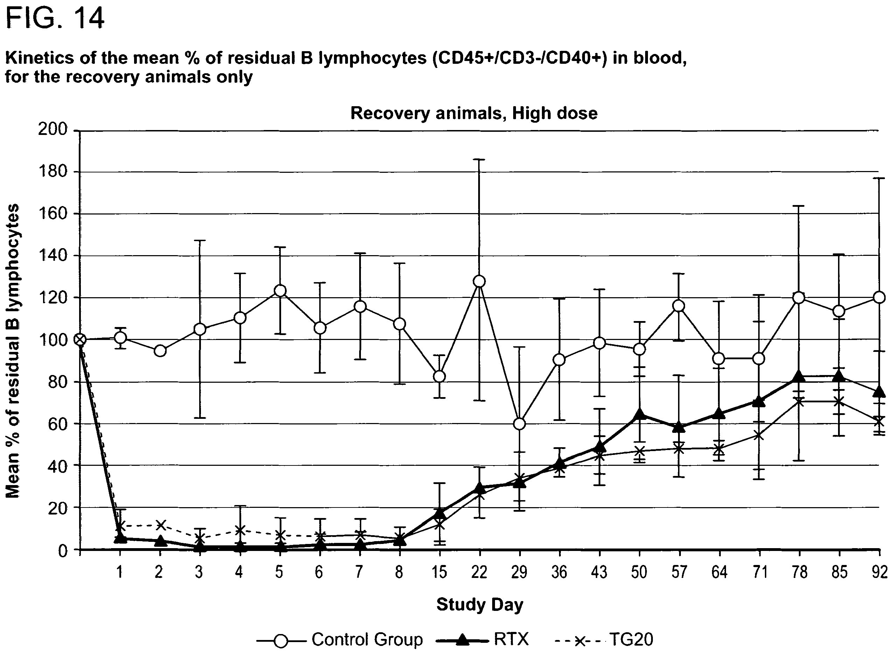

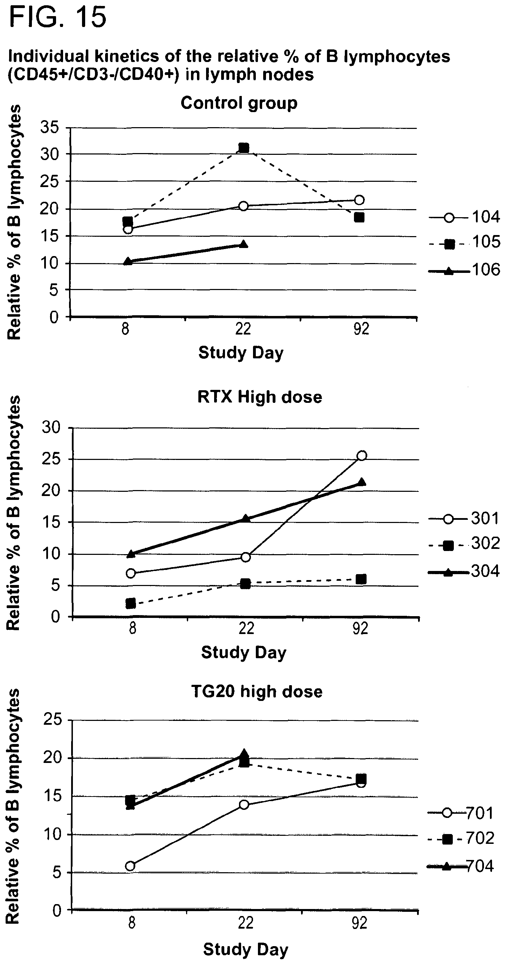

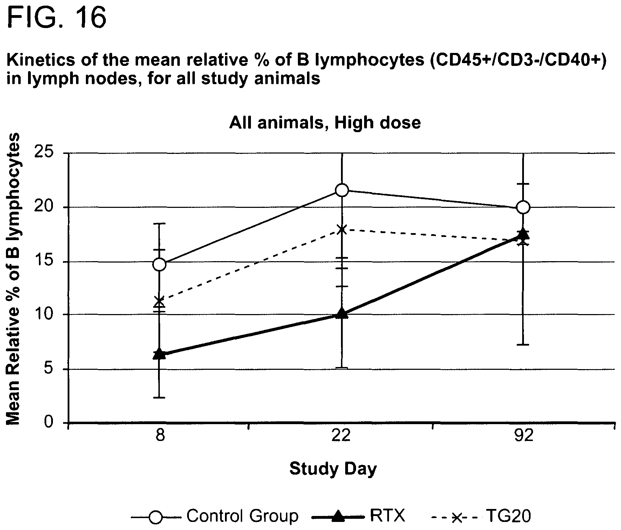

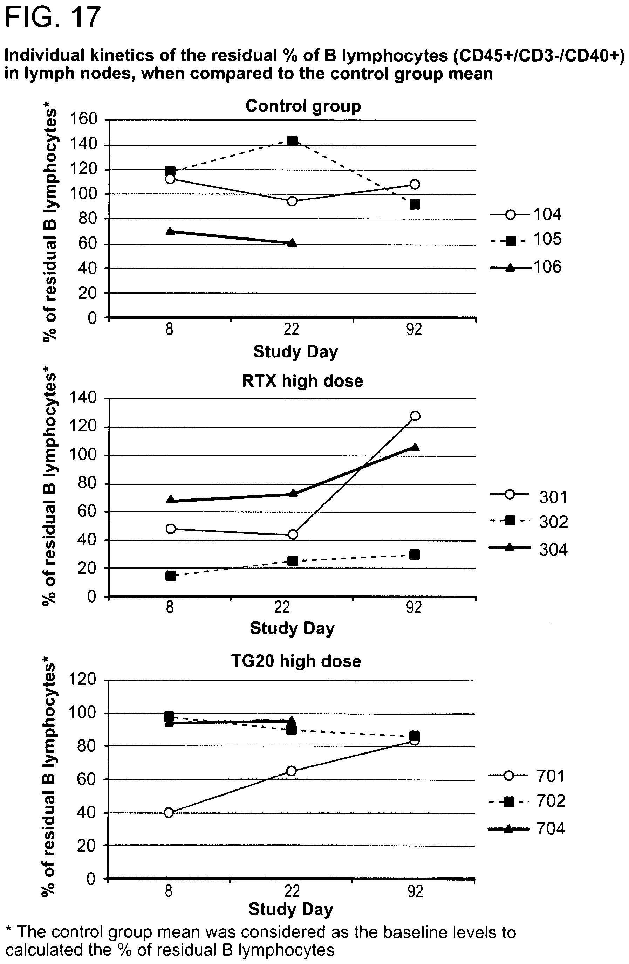

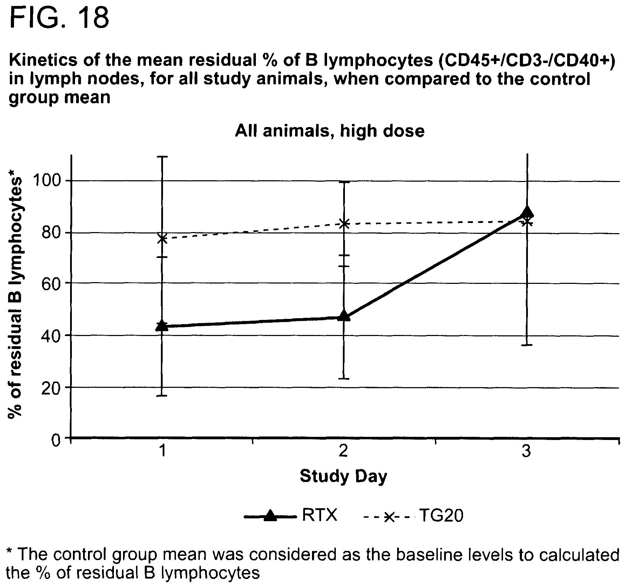

In conclusion, administration of different anti-CD20 antibodies, RTX, and TG20, by a single intravenous bolus injection at two different doses, 0.03 and 0.3 mg/kg/day, was clinically well tolerated in monkeys at both dose levels. A test article-related dose-dependent depletion of the blood and lymph node B lymphocytes was observed with both compounds. Slight differences were noted between the compounds in terms of extent and incidence of B lymphocyte depletion and rapidity of recovery. At the low dose, the extent of B lymphocyte depletion was lower with TG20. At the high dose, the incidence of full B lymphocytes depletion was greater with RTX and lower with TG20. At all doses, the recovery was quicker with RTX than with TG20. A similar test article-related decrease of blood NK lymphocytes was observed with both compounds with recovery by Day 22. A test article-related decrease of the blood total T lymphocytes was also observed with RTX (high dose only), but not with TG20. The decrease was slight in magnitude and quickly recovered on Day 3, for most of the animals (See **Figures 12-18**)

## Claims

1. A method for producing a highly galactosylated population of antibodies, comprising:
   transgenically expressing a population of antibodies in mammary gland epithelial cells of a goat such that a highly galactosylated population of antibodies is produced, wherein the level of galactosylation of the antibodies in the population is at least 70%.

2. The method of claim 1, wherein the method further comprises collecting the population of antibodies produced.

3. The method of claim 1 or 2, wherein the level of galactosylation of the antibodies in the population is at least 80%.

4. The method of claim 4, wherein the level of galactosylation of the antibodies in the population is at least 90%.

5. The method of claim 1 or 2, wherein the level of fucosylation of the antibodies in the population is at least 80%.

6. The method of claim 5, wherein the level of fucosylation of the antibodies in the population is at least 90%.

7. The method of claim 1 or 2, wherein the ratio of the level of galactosylation of the antibodies in the population to the level of fucosylation of the antibodies in the population is between 0.8 and 1.2.

8. The method of any one of claims 1 to 7, wherein:

   i) the population of antibodies comprises antibodies that comprise mono-galactosylated N-glycans;
   ii) the population of antibodies comprises antibodies that comprise bi-galactosylated N-glycans;
   iii) the population of antibodies comprises antibodies that comprise both mono-galactosylated N-glycans and bi-galactosylated N-glycans; or
   iv) at least 35% of the antibodies in the population comprise bi-galactosylated N-glycans and at least 5% of the antibodies in the population comprise mono-galactosylated N-glycans.

9. The method of any one of claims 1 to 8, wherein the antibodies in the population are directed to the same antigen epitope.

10. The method of any one of claims 1 to 9, wherein the antibodies in the population are anti-CD20 antibodies.

11. The method of any of claims 1 to 10, wherein the light chain and heavy chain of the antibodies in the population comprise amino acid sequences as set forth in SEQ ID NO:1 and SEQ ID NO:2.

## Patentansprüche

1. Ein Verfahren zur Herstellung einer hochgradig galaktosylierten Antikörperpopulation, umfassend:

transgene Expression einer Antikörperpopulation in Milchdrüsenepithelzellen einer Ziege, so dass eine hochgradig galaktosylierte Antikörperpopulation hergestellt wird, wobei der Galaktosylierungsgrad der Antikörper in der Population mindestens 70% beträgt.

**2.** Das Verfahren nach Anspruch 1, wobei das Verfahren ferner das Einsammeln der hergestellten Antikörperpopulation umfasst.

**3.** Das Verfahren nach Anspruch 1 oder 2, wobei der Galaktosylierungsgrad der Antikörper in der Population mindestens 80% beträgt.

**4.** Das Verfahren nach Anspruch 4, wobei der Galaktosylierungsgrad der Antikörper in der Population mindestens 90% beträgt.

**5.** Das Verfahren nach Anspruch 1 oder 2, wobei der Fucosylierungsgrad der Antikörper in der Population mindestens 80% beträgt.

**6.** Das Verfahren nach Anspruch 5, wobei der Fucosylierungsgrad der Antikörper in der Population mindestens 90% beträgt.

**7.** Das Verfahren nach Anspruch 1 oder 2, wobei das Verhältnis des Galaktosylierungsgrades der Antikörper in der Population zum Fucosylierungsgrad der Antikörper in der Population zwischen 0,8 und 1,2 liegt.

**8.** Das Verfahren nach einem der Ansprüche 1 bis 7, wobei:

i) die Antikörperpopulation Antikörper umfasst, die mono-galaktosylierte N-Glykane umfassen;
ii) die Antikörperpopulation Antikörper umfasst, die bi-galaktosylierte N-Glykane umfassen;
iii) die Antikörperpopulation Antikörper umfasst, die sowohl mono-galaktosylierte N-Glykane als auch bi-galaktosylierte N-Glykane umfassen; oder
iv) mindestens 35% der Antikörper in der Population bi-galaktosylierte N-Glykane umfassen und mindestens 5% der Antikörper in der Population mono-galaktosylierte N-Glykane umfassen.

**9.** Das Verfahren nach einem der Ansprüche 1 bis 8, wobei die Antikörper in der Population gegen das gleiche Antigen-Epitop gerichtet sind.

**10.** Das Verfahren nach einem der Ansprüche 1 bis 9, wobei die Antikörper in der Population Anti-CD20-Antikörper sind.

**11.** Das Verfahren nach einem der Ansprüche 1 bis 10, wobei die leichte Kette und die schwere Kette der Antikörper in der Population Aminosäuresequenzen wie in SEQ ID NO:1 und SEQ ID NO:2 angeführt umfassen.

**Revendications**

**1.** Méthode de production d'une population fortement galactosylée d'anticorps, comprenant : l'expression transgénique d'une population d'anticorps dans des cellules épithéliales de glande mammaire de chèvre de façon qu'une population fortement galactosylée d'anticorps soit produite, dans laquelle le niveau de galactosylation des anticorps dans la population est d'au moins 70 %.

**2.** Méthode selon la revendication 1, laquelle méthode comprend en outre la collecte de la population d'anticorps produite.

**3.** Méthode selon la revendication 1 ou 2, dans laquelle le niveau de galactosylation des anticorps dans la population est d'au moins 80 %.

**4.** Méthode selon la revendication 4, dans laquelle le niveau de galactosylation des anticorps dans la population est d'au moins 90 %.

**5.** Méthode selon la revendication 1 ou 2, dans laquelle le niveau de fucosylation des anticorps dans la population est d'au moins 80 %.

**6.** Méthode selon la revendication 5, dans laquelle le niveau de fucosylation des anticorps dans la population est d'au moins 90 %.

**7.** Méthode selon la revendication 1 ou 2, dans laquelle le rapport du niveau de galactosylation des anticorps dans la population au niveau de fucosylation des anticorps dans la population est compris entre 0,8 et 1,2.

**8.** Méthode selon l'une quelconque des revendications 1 à 7, dans laquelle :

i) la population d'anticorps comprend des anticorps qui comprennent des N-glycanes mono-galactosylés ;
ii) la population d'anticorps comprend des anticorps qui comprennent des N-glycanes bi-galactosylés ;
iii) la population d'anticorps comprend des anticorps qui comprennent à la fois des N-glycanes mono-galactosylés et des N-glycanes bi-galactosylés ; ou
iv) au moins 35 % des anticorps dans la population comprennent des N-glycanes bi-galactosylés et au moins 5 % des anticorps dans la population comprennent des N-glycanes mono-galactosylés.

**9.** Méthode selon l'une quelconque des revendications 1 à 8, dans laquelle les anticorps dans la population sont dirigés vers le même épitope antigénique.

**10.** Méthode selon l'une quelconque des revendications 1 à 9, dans laquelle les anticorps dans la population sont des anticorps anti-CD20.

**11.** Méthode selon l'une quelconque des revendications 1 à 10, dans laquelle la chaîne légère et la chaîne lourde des anticorps dans la population comprennent des séquences d'acides aminés telles qu'indiquées dans la SEQ ID NO : 1 et la SEQ ID NO : 2.

# FIG. 1A

**Tg20 Light Chain (SEQ ID NO:1)**

```
Gln Ile Val Leu Ser Gln Ser Pro Ala Ile Leu Ser Ala Ser Pro Gly
1               5               10              15

Glu Lys Val Thr Met Thr Cys Arg Ala Ser Ser Ser Val Ser Tyr Met
            20              25              30

His Trp Tyr Gln Gln Lys Pro Gly Ser Ser Pro Lys Pro Trp Ile Tyr
        35              40              45

Ala Thr Ser Asn Leu Ala Ser Gly Val Pro Ala Arg Phe Ser Gly Ser
    50              55              60

Gly Ser Gly Thr Ser Tyr Ser Phe Thr Ile Ser Arg Val Glu Ala Glu
65              70              75              80

Asp Ala Ala Thr Tyr Tyr Cys Gln Gln Trp Thr Phe Asn Pro Pro Thr
            85              90              95

Phe Gly Gly Gly Thr Arg Leu Glu Ile Lys Arg Thr Val Ala Ala Pro
            100             105             110

Ser Val Phe Ile Phe Pro Pro Ser Asp Glu Gln Leu Lys Ser Gly Thr
        115             120             125

Ala Ser Val Val Cys Leu Leu Asn Asn Phe Tyr Pro Arg Glu Ala Lys
    130             135             140

Val Gln Trp Lys Val Asp Asn Ala Leu Gln Ser Gly Asn Ser Gln Glu
145             150             155             160

Ser Val Thr Glu Gln Asp Ser Lys Asp Ser Thr Tyr Ser Leu Ser Ser
            165             170             175

Thr Leu Thr Leu Ser Lys Ala Asp Tyr Glu Lys His Lys Val Tyr Ala
            180             185             190

Cys Glu Val Thr His Gln Gly Leu Ser Ser Pro Val Thr Lys Ser Phe
        195             200             205

Asn Arg Gly Glu Cys
        210
```

# FIG. 1B

**Tg20 Heavy Chain (SEQ ID NO:2)**

```
Gln Ala Tyr Leu Gln Gln Ser Gly Ala Glu Leu Val Arg Pro Gly Ala
1               5               10                      15

Ser Val Lys Met Ser Cys Lys Ala Ser Gly Tyr Thr Phe Thr Ser Tyr
            20              25                  30

Asn Met His Trp Val Lys Gln Thr Pro Arg Gln Gly Leu Glu Trp Ile
        35                  40                  45

Gly Gly Ile Tyr Pro Gly Asn Gly Asp Thr Ser Tyr Asn Gln Lys Phe
    50              55                  60

Lys Gly Lys Ala Thr Leu Thr Val Gly Lys Ser Ser Ser Thr Ala Tyr
65              70                  75                      80

Met Gln Leu Ser Ser Leu Thr Ser Glu Asp Ser Ala Val Tyr Phe Cys
            85                  90                  95

Ala Arg Tyr Asp Tyr Asn Tyr Ala Met Asp Tyr Trp Gly Gln Gly Thr
            100             105                 110

Ser Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130             135                 140

Cys Leu Val Lys Asp Tyr Phe Pro Glu Pro Val Thr Val Ser Trp Asn
145             150                 155                     160

Ser Gly Ala Leu Thr Ser Gly Val His Thr Phe Pro Ala Val Leu Gln
            165                 170                 175

Ser Ser Gly Leu Tyr Ser Leu Ser Ser Val Val Thr Val Pro Ser Ser
        180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210             215                 220

His Thr Cys Pro Pro Cys Pro Ala Pro Glu Leu Leu Gly Gly Pro Ser
225             230                 235                     240

Val Phe Leu Phe Pro Pro Lys Pro Lys Asp Thr Leu Met Ile Ser Arg
            245                 250                 255

Thr Pro Glu Val Thr Cys Val Val Val Asp Val Ser His Glu Asp Pro
            260             265                 270
```

# FIG. 1B-Continuation

## Tg20 Heavy Chain -continuation (SEQ ID NO:2)

```
Glu Val Lys Phe Asn Trp Tyr Val Asp Gly Val Glu Val His Asn Ala
        275             280             285

Lys Thr Lys Pro Arg Glu Glu Gln Tyr Asn Ser Thr Tyr Arg Val Val
    290             295             300

Ser Val Leu Thr Val Leu His Gln Asp Trp Leu Asn Gly Lys Glu Tyr
305             310             315             320

Lys Cys Lys Val Ser Asn Lys Ala Leu Pro Ala Pro Ile Glu Lys Thr
            325             330             335

Ile Ser Lys Ala Lys Gly Gln Pro Arg Glu Pro Gln Val Tyr Thr Leu
            340             345             350

Pro Pro Ser Arg Asp Glu Leu Thr Lys Asn Gln Val Ser Leu Thr Cys
            355             360             365

Leu Val Lys Gly Phe Tyr Pro Ser Asp Ile Ala Val Glu Trp Glu Ser
    370             375             380

Asn Gly Gln Pro Glu Asn Asn Tyr Lys Thr Thr Pro Pro Val Leu Asp
385             390             395             400

Ser Asp Gly Ser Phe Phe Leu Tyr Ser Lys Leu Thr Val Asp Lys Ser
            405             410             415

Arg Trp Gln Gln Gly Asn Val Phe Ser Cys Ser Val Met His Glu Ala
            420             425             430

Leu His Asn His Tyr Thr Gln Lys Ser Leu Ser Leu Ser Pro Gly Lys
        435             440             445
```

FIG. 2

TG20 Construct

**Ref. is Low fucose reference antibody**

## FIG. 3

FIG. 4

EP 2 741 769 B1

FIG. 5

Principle:

| | | | | |
|---|---|---|---|---|
| Whole blood | + | Anti-CD20 | 18h incubation $\longrightarrow$ | Measurement of B cell depletion |

1 - ADCC
Cytotoxicity mediated by the NK cells

2 - CDC
Cytotoxicity mediated by the complement

FIG. 6

EP 2 741 769 B1

**Generation of TG20 Expressing Goats**

| Founder Goat | Transgenes Copy# | FISH | Induced Lactation Expression Level | Natural Lactation Expression Level |
|---|---|---|---|---|
| L0694 | High (> 10 copies) | C25 (?) | NA | NA |
| L0716 | 4-5 copies | C14 | > 10 g/l | 10 g/l |
| L0737 | 1-2 copies | X | > 2 g/l | 2.5 g/l |
| L0762 | 4-5 copies | C19 | NA | ~ 4 |
| L0778 | 1-2 copies | C26 | 5 g/l | 4-5 g/l |
| **L0824, L0825** | **3-4 copies** | **C14q1** | **2 g/l** | **2 g/l** |
| L0842 | 5 copies | C3 | NA | 11-15 g/l |

FIG. 7

| Functional Analysis of TG20 | | | | |
|---|---|---|---|---|
| | CD16 activity | CD16 binding | CDC | ADCC |
| **Tg20** | 100% | 100% | 100% | 100% |
| **Rituxan** | < 2% | Not detectable | 59% | 6% |

# FIG. 8

## FIG. 9

CD16 assay

Antibody concentration (ng/mL)
ADCC mean n = 3 donors in duplicate

- ■— RTX lot810598
- △- TG20

**Pharmacokinetic Profile in Cynomolgus monkeys**

| PK Parameters | Unit | RTX 0.375 mg/kg | | TG20 0.255 mg/kg | |
|---|---|---|---|---|---|
| | | **#301** | **#304** | **#701** | **#704** |
| | | | | | |
| t1/2 elim | hr | 71 | 76 | 58 | 95 |
| Cmax | ng/ml | 8462 | 7387 | 6841 | 5174 |
| AUC (0-168h)/Dose | hr*kg*ng/mL/mg | 601106 | 331930 | 478180 | 278517 |
| AUC (0-inf)/Dose | hr*kg*ng/mL/mg | 672871 | 396415 | 500679 | 303662 |
| Cl | ml/h/kg | 1.49 | 2.52 | 1.99 | 3.29 |

FIG. 10

**Pharmacological Activity in Cynomolgus monkeys**

FIG. 11

# FIG. 12A

**Individual kinetics of the % of residual B lymphocytes
(CD45+/CD3-/CD40+) in blood**

Control group

RTX low dose

RTX high dose

# FIG. 12B

**Individual kinetics of the % of residual B lymphocytes (CD45+/CD3-/CD40+) in blood**

TG20 low dose

TG20 high dose

# FIG. 13

**Kinetics of the mean % of residual B lymphocytes
(CD45+/CD3-/CD40+) in blood, for all study animals**

All animals, Low dose

All animals, High dose

# FIG. 14

**Kinetics of the mean % of residual B lymphocytes (CD45+/CD3-/CD40+) in blood, for the recovery animals only**

Recovery animals, High dose

—O— Control Group    —▲— RTX    - -x- - TG20

EP 2 741 769 B1

# FIG. 15

**Individual kinetics of the relative % of B lymphocytes (CD45+/CD3-/CD40+) in lymph nodes**

Control group

RTX High dose

TG20 high dose

# FIG. 16

**Kinetics of the mean relative % of B lymphocytes (CD45+/CD3-/CD40+) in lymph nodes, for all study animals**

All animals, High dose

—O— Control Group ——▲—— RTX - - ✕- - TG20

# FIG. 17

**Individual kinetics of the residual % of B lymphocytes (CD45+/CD3-/CD40+) in lymph nodes, when compared to the control group mean**

* The control group mean was considered as the baseline levels to calculated the % of residual B lymphocytes

# FIG. 18

**Kinetics of the mean residual % of B lymphocytes (CD45+/CD3-/CD40+) in lymph nodes, for all study animals, when compared to the control group mean**

All animals, high dose

* The control group mean was considered as the baseline levels to calculated the % of residual B lymphocytes

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 2861080
- WO 2007048077 A
- US 20060057638 A
- US 20060127950 A
- US 5591669 A
- US 5598369 A
- US 5545806 A
- US 5545807 A
- US 6150584 A
- US 5945577 A
- US 5284656 A, Platz
- US 5451569 A, Wong

### Non-patent literature cited in the description

- **REFF M. E.** *Blood,* 1994, vol. 83, 435
- **ROBAK et al.** *Biodrugs,* 2011, vol. 25, 13-25
- **WALDMANN.** *Science,* 1991, vol. 252, 1657
- **DITULLIO.** *BIOTECHNOLOGY,* 1992, vol. 10, 74-77
- **GORDON et al.** *BIOTECHNOLOGY,* 1987, vol. 5, 1183-1187
- **CLARK et al.** *BIOTECHNOLOGY,* 1989, vol. 7, 487-492
- **SOULIER et al.** *FEBS LETTS.,* 1992, vol. 297, 13
- **POLLOCK et al.** *Journal of Immunological Methods,* 10 December 1999, vol. 231 (1-2), 147-157
- Soluble Polymer-Enzyme Adducts. **ABUCHOWSKI ; DAVIS.** Enzymes as Drugs. Wiley-Interscience, 1981, 367-383
- **NEWMARK et al.** *J. Appl. Biochem.,* 1982, vol. 4, 185-189
- **ADJEI et al.** *Pharmaceutical Research,* 1990, vol. 7, 565-569
- **ADJEI et al.** *International Journal of Pharmaceutics,* 1990, vol. 63, 135-144
- **BRAQUET et al.** *Journal of Cardiovascular Pharmacology,* 1989, vol. 13 (5), 143-146
- **HUBBARD et al.** *Annals of Internal Medicine,* 1989, vol. III, 206-212
- **SMITH et al.** *J. Clin. Invest.,* 1989, vol. 84, 1145-1146
- **OSWEIN et al.** Aerosolization of Proteins. *Proceedings of Symposium on Respiratory Drug Delivery II,* March 1990
- **DEBS et al.** *J. Immunol.,* 1988, vol. 140, 3482-3488
- **LANGER.** *Science,* 1990, vol. 249, 1527-1533
- **H.S. SAWHNEY ; C.P. PATHAK ; J.A. HUBELL.** *Macromolecules,* 1993, vol. 26, 581-587
- **MELICAN et al.** *Theriogenology,* 2005, vol. 63, 1549
- **EBERT et al.** *Biotech,* 1994, vol. 12, 699-702
- **GUILE GR ; RUDD PM ; WING DR ; PRIME SB ; DWEK RA.** A rapid high-resolution high-performance liquid chromatographic method for separating glycan mixtures and analyzing oligosaccharide profiles. *Anal Biochem.,* 05 September 1996, vol. 240 (2), 210-26
- **PACKER NH ; LAWSON MA ; JARDINE DR ; REDMOND JW.** A general approach to desalting oligosaccharides released from glycoproteins. *Glycoconj J.,* August 1998, vol. 15 (8), 737-47
- **VIVIER E et al.** *Int. Immunol.,* 1992, vol. 4 (11), 1313-1323
- **O'BRIEN, J. et al.** *Eur. J. Biochem.,* 2000, vol. 267, 5421-5426